# EUROPEAN PATENT APPLICATION

(11) **EP 4 166 159 A1**
(43) Date of publication of application: **19.04.2023**
(21) Application number: 21820830.4
(22) Date of filing: 09.06.2021
(51) Int. Cl.: A61K 39/395, C07K 16/24, A61K 39/00, A61P 37/02

(54) **PHARMACEUTICAL COMPOSITION OF ANTI-IL-17A ANTIBODIES AND USE THEREOF**

(30) Priority: 10.06.2020 CN 202010524523
(71) Applicant: Shanghai Junshi Biosciences Co., Ltd., Pilot Free Trade Zone Shanghai 201210 (CN); Suzhou Junmeng Biosciences Co., Ltd., Suzhou, Jiangsu 215002 (CN); Shanghai Junshi Biotechnology Co., Ltd., Shanghai 201400 (CN)
(72) Inventor: LIU, Hongchuan, Suzhou, Jiangsu 215002 (CN); LIU, Peixiang, Suzhou, Jiangsu 215002 (CN); ZHANG, Jing, Suzhou, Jiangsu 215002 (CN); WANG, Jing, Suzhou, Jiangsu 215002 (CN); LIU, Hui, Suzhou, Jiangsu 215002 (CN); LI, Yuanyuan, Suzhou, Jiangsu 215002 (CN); FENG, Hui, Suzhou, Jiangsu 215002 (CN); YAO, Sheng, Suzhou, Jiangsu 215002 (CN)
(74) Representative: V.O.
(86) International application number: PCT/CN2021/099257
(87) International publication number: WO 2021/249445

(57) **Abstract**

Provided are a stable pharmaceutical composition of an anti-IL-17A antibody and an application thereof in medicine. The pharmaceutical composition contains an anti-IL-17A antibody or an antigen-binding fragment thereof, and a buffer, can further contain at least one stabilizer, and can optionally further contain a surfactant.

## Description

### Technical Field

The present invention relates to the field of therapeutic pharmaceutical compositions. In particular, the present invention relates to the field of pharmaceutical formulations containing a humanized antibody that specifically binds to IL-17A.

### Background Art

Interleukin 17 (IL-17), also known as CTLA-8 or IL-17A, plays a key coordinating role in the immune system. A total of six members in the IL-17 family, including IL-17A, IL-17B, IL-17C, IL-17D, IL-17E, and IL-17F, all comprise four crucial and highly conserved cysteine residues. However, these members have large differences in biological effects, and IL-17A and IL-17F are the closest in homology and biological function and are currently the most intensively studied. IL-17A expressed *in vivo* has a 23-amino acid N-terminal signal peptide that would be cleaved to give mature IL-17A. The mature IL-17A is linked by disulfide bonds, and is generally secreted and exists in the form of a homodimer, and sometimes the mature IL-17A is also linked with IL-17F to form a heterodimer IL-17AF. Generally, IL-17A or IL-17 refers to an IL-17A homodimeric protein, which is mainly produced by T helper 17 (Th17), and can also be synthesized and secreted by other immune cells such as γ δ T cells, LTi (Lymphoid Tissue inducer cells), ILCs (Innate Lymphoid Cells) and NKT (Natural Killer T) cells. The regulation of IL-17A expression is very complicated. Studies have found that cytokines such as IL-6, IL-1 β and TGF β can induce naive CD4+ T cells to differentiate into Th17, but at this time Th17 cells are less stable and secrete a small amount of IL-17A which has a low tissue damaging effect. When present, IL-23 causes inflammatory outbreaks and tissue damage by promoting Th17 cell stability and sustained secretion of IL-17A, upregulating expression of pro-inflammatory factors (IL-22, CSF-2 and IFN-γ) and downregulating expression of anti-inflammatory factors (IL-2, IL-27 and IL-12) and the like. Therefore, when IL-23 is abnormally expressed in tissues, the IL-17A pathway plays a key role in tissue damage.

IL-17 is generally secreted at a specific site, and binds to the IL-17 receptor (IL-17R) on the surface of target cells to exert its effects in local tissues. The IL-17R family includes five members, IL-17RA, IL-17RB, IL-17RC, IL-17RD and IL-17RE, which are widely expressed on the membrane of a variety of cells. IL-17 mainly binds to the IL-17RA/IL-17RC complex on the surface of cells of non-hematopoietic origin (such as epithelial cells and mesenchymal cells) to exert its effects which are promoting the secretion of cytokines (such as IL-6, G-CSF, GM-CSF, IL-10, TGF-β and TNF-α), chemokines (including IL-8, CXCL1 and MCP-1), and prostaglandins (such as PGE2) by cells, and inducing neutrophils and macrophages to aggregate and release reactive oxygen species (ROS) which damage tissues.

Autoimmune diseases, such as psoriasis, rheumatoid arthritis, ankylosing spondylitis, Crohn's disease and multiple sclerosis, seriously threaten human health. Studies have found that the secretion disorder of IL-17 is closely related to the occurrence and development of such diseases. Antibodies targeting IL-17 can effectively alleviate the symptoms of autoimmune diseases by inhibiting the IL-17-IL-17R signaling pathway. Cosentyx (secukinumab) developed by Novartis is the first monoclonal antibody against IL-17 around the world, and the approved indications include moderate to severe plaque psoriasis. Cosentyx provides an important first-line biologic treatment option for a wide range of population suffering from psoriasis. However, there is an urgent need and an important significance to develop different anti-IL-17 antibodies with different structures, better efficacy and broader indications to treat autoimmune-related conditions such as psoriasis, rheumatoid arthritis and ankylosing spondylitis, and other IL-17-related diseases.

### Summary of the Invention

The pharmaceutical composition of the present invention is a highly stable pharmaceutical composition comprising a humanized antibody that specifically binds to IL-17A. In particular, the present invention finds that the combination of arginine hydrochloride and sucrose has the unexpected properties, i.e., high stability and low viscosity.

The present invention provides a pharmaceutical composition, comprising: (1) a buffer; (2) an anti-IL-17A antibody or an antigen-binding fragment thereof.

In some embodiments, for the anti-IL-17A antibody or the antigen-binding fragment thereof described above, HCDR1 is selected from SEQ ID NOs: 1, 7 and 13; HCDR2 is selected from 2, 8 and 14; HCDR3 is selected from 3, 9 and 15; LCDR1 is selected from 4, 10 and 16; LCDR2 is selected from 5, 11 and 17; and LCDR3 is selected from 6, 12 and 18.

In some embodiments, for the anti-IL-17A antibodis described above, VH is selected from SEQ ID NOs: 19, 21, 23 and 26, and VL is selected from SEQ ID NOs: 20, 22, 24, 25 and 27.

In some embodiments, for the anti-IL-17A antibody described above, HC is selected from SEQ ID NOs: 28, 30, 32 and 35; and LC is selected from SEQ ID NOs: 29, 31, 33, 34 and 36.

In some embodiments, the anti-IL-17A antibody or the antigen-binding fragment thereof described above has HCDR1, HCDR2 and HCDR3 as set forth in SEQ ID NO: 1, SEQ ID NO: 2 and SEQ ID NO: 3, respectively, and LCDR1, LCDR2 and LCDR3 as set forth in SEQ ID NO: 4, SEQ ID NO: 5 and SEQ ID NO: 6, respectively.

In some embodiments, the anti-IL-17A antibody or the antigen-binding fragment thereof described above has HCDR1, HCDR2 and HCDR3 as set forth in SEQ ID NO: 7, SEQ ID NO: 8 and SEQ ID NO: 9, respectively, and LCDR1, LCDR2 and LCDR3 as set forth in SEQ ID NO: 10, SEQ ID NO: 11 and SEQ ID NO: 12, respectively.

In some embodiments, the anti-IL-17A antibody or the antigen-binding fragment thereof described above has HCDR1, HCDR2 and HCDR3 as set forth in SEQ ID NO: 13, SEQ ID NO: 14 and SEQ ID NO: 15, respectively, and LCDR1, LCDR2 and LCDR3 as set forth in SEQ ID NO: 16, SEQ ID NO: 17 and SEQ ID NO: 18, respectively.

In some embodiments, the anti-IL-17A antibody or the antigen-binding fragment thereof in the pharmaceutical composition has a concentration of about 1-300 mg/mL, preferably about 10-250 mg/mL, preferably about 20-200 mg/mL, preferably about 60-180 mg/mL, more preferably about 80-150 mg/mL; More preferably, the anti-IL-17A antibody or the antigen-binding fragment thereof described above has a concentration of about 20 mg/mL, 30 mg/mL, 40 mg/mL, 50 mg/mL, 60 mg/mL, 70 mg/mL, 80 mg /mL, 90 mg/mL, 100 mg/mL, 110 mg/mL, 120 mg/mL, 130 mg/mL, 140 mg/mL, 150 mg/mL, 160 mg/mL, 170 mg/mL, 180 mg/mL or 200 mg/mL, preferably about 80 mg/mL or 150 mg/mL.

In some embodiments, the buffer described above is selected from one or more of acetate buffer, citrate buffer, succinate buffer and histidine buffer.

In some embodiments, the buffer described above is histidine buffer, preferably, the histidine buffer is selected from histidine-hydrochloride buffer or histidine-acetate buffer, preferably histidine-hydrochloride buffer.

In some embodiments, the histidine-hydrochloride buffer described above are made from histidine and histidine hydrochloride, preferably L-histidine and L-histidine monohydrochloride. In some embodiments, the histidine buffer is made from 1-30 mM of L-histidine and 1-30 mM of L-histidine monohydrochloride. In some embodiments, the histidine buffer is made from histidine and histidine hydrochloride in a molar ratio of 1 : 1 to 1 : 4. In some embodiments, the histidine buffer is made from histidine and histidine hydrochloride in a molar ratio of 1 : 1. In some embodiments, the histidine buffer is made from histidine and histidine hydrochloride in a molar ratio of 1 : 3. In some embodiments, the histidine formulation is: a histidine buffer with pH 5.5 made from 4.5 mM of L-histidine and 15.5 mM of L-histidine monohydrochloride. In some embodiments, the histidine formulation is: a histidine buffer with pH 5.5 made from 7.5 mM of L-histidine and 22.5 mM of L-histidine monohydrochloride. In some embodiments, the histidine formulation is: a histidine buffer with pH 6.0 made from 15 mM of histidine and 15 mM of histidine hydrochloride.

In some embodiments, the histidine buffer described above is a histidine-acetate buffer, preferably, the molar ratio of histidine to acetate is 1 : 1 to 1.5 : 1, preferably, such a buffer has a pH of 5.5 ± 0.3, preferably about 5.5, preferably such a buffer contains 15-20 mM of histidine and 12-15 mM of acetic acid.

In some embodiments, the buffer described above is an acetate buffer, preferably, the acetate buffer is an acetic acid-sodium acetate buffer or an acetic acid-potassium acetate buffer, preferably an acetic acid-sodium acetate buffer.

In some embodiments, the buffer described above is a citrate buffer, preferably, the citrate buffer is a citric acid-sodium citrate buffer.

In some embodiments, the buffer described above is a succinate buffer, preferably, the succinate buffer is a succinic acid-sodium succinate buffer.

In some embodiments, the buffer described above has a concentration of about 1-200 mM, preferably about 5-200 mM, preferably about 10-50 mM, preferably about 10-30 mM; and preferably about 10-20 mM, and non-limiting examples of the concentration of the buffer described above are about 5 mM, 10 mM, 15 mM, 20 mM, 25 mM, 30 mM, 40 mM, 45 mM, 50 mM, 60 mM, 70 mM, 80 mM, 90 mM, 100 mM, 105 mM, 110 mM, 115 mM, 120 mM, 130 mM, 140 mM, 150 mM, 160 mM, 170 mM or 180 mM or a range formed by any two values within these ranges as endpoints, preferably 10 mM, 15 mM, 20 mM or 30 mM.

In some embodiments, the buffer described above has a pH of about 5.0-6.5, preferably about 5.0-6.0, preferably about 5.5-6.5, preferably about 5.0-5.5, preferably about 5.5-6.0, preferably about 6.0-6.5, and non-limiting examples of the pH of the buffer described above are about 5.0, 5.1, 5.2, 5.3, 5.4, 5.5, 5.6, 5.7, 5.8, 5.9, 6.0, 6.1, 6.2, 6.3, 6.4 and 6.5, preferably about 5.0, 5.5 or 6.0.

In some embodiments, the pharmaceutical composition described above further comprises a stabilizer selected from one or more of arginine hydrochloride, proline, glycine, sodium chloride, mannitol, sorbitol, sucrose, maltose, xylitol and trehalose.

In some embodiments, the stabilizer described above has a concentration of about 10 mM to 400 mM, preferably 20 mM-300 mM, and more preferably 30 mM-200 mM.

In some embodiments, the stabilizer described above is sodium chloride at a concentration of about 30-200 mM; or the stabilizer is mannitol at a concentration of about 100-300 mM; or the stabilizer is sorbitol at a concentration of about 100-300 mM; or the stabilizer is sucrose at a concentration of about 100-300 mM; or the stabilizer is trehalose at a concentration of about 100-300 mM; or the stabilizer is arginine hydrochloride at a concentration of about 30-200 mM; or the stabilizer is proline at a concentration of about 100-300 mM; or the stabilizer is glycine at a concentration of about 100-300 mM.

In some embodiments, the stabilizer described above is a combination of about 30-200 mM of sodium chloride and about 30-200 mM of mannitol; or the stabilizer is a combination of about 30-200 mM of sodium chloride and about 30-200 mM of sorbitol; or the stabilizer is a combination of about 30-200 mM of sodium chloride and about 30-200 mM of sucrose; or the stabilizer is a combination of about 30-200 mM of arginine hydrochloride and about 30-200 mM of mannitol; or the stabilizer is a combination of about 30-200 mM of arginine hydrochloride and about 30-200 mM of sucrose; or the stabilizer is a combination of about 30-200 mM of arginine hydrochloride and about 30-200 mM of sorbitol; or the stabilizer is a combination of about 30-200 mM of arginine hydrochloride and about 30-200 mM of trehalose.

In some embodiments, the stabilizer described above is sodium chloride. In some embodiments, the stabilizer described above is sodium chloride at a concentration of about 30-200 mM, the concentration of the sodium chloride described above is preferably about 50-190 mM, preferably about 100-180 mM, preferably about 120-170 mM, and preferably about 130-150 mM, and non-limiting examples of the concentration of the sodium chloride described above are about 100 mM, 110 mM, 120 mM, 125 mM, 130 mM, 135 mM, 140 mM, 145 mM, 150 mM, 155 mM , 160 mM, 170 mM, 180 mM, 190 mM and 200 mM, preferably 135 mM or 140 mM.

In some embodiments, the stabilizer described above is mannitol. In some embodiments, the stabilizer described above is mannitol at a concentration of about 100-300 mM, the concentration of the mannitol described above is preferably about 150-300 mM, and preferably about 200-280 mM, and non-limiting examples of the concentration of the mannitol described above are about 200 mM, 210 mM, 220 mM, 230 mM, 240 mM, 250 mM, 260 mM, 270 mM and 280 mM, preferably 240 mM.

In some embodiments, the stabilizer described above is sorbitol. In some embodiments, the stabilizer described above is sorbitol at a concentration of about 100-300 mM, the concentration of the sorbitol described above is preferably about 150-300 mM, preferably about 200-280 mM, and non-limiting examples of the concentration of the sorbitol described above are about 200 mM, 210 mM, 220 mM, 230 mM, 240 mM, 250 mM, 260 mM, 270 mM and 280 mM, preferably 240 mM.

In some embodiments, the stabilizer described above is sucrose. In some embodiments, the stabilizer described above is sucrose at a concentration of about 100-300 mM, the concentration of the sucrose described above is preferably about 150-300 mM, and preferably about 200-280 mM, and non-limiting examples of the concentration of the sucrose described above are about 200 mM, 210 mM, 220 mM, 230 mM, 240 mM, 250 mM, 260 mM, 270 mM and 280 mM, preferably 220 mM.

In some embodiments, the stabilizer described above is trehalose. In some embodiments, the stabilizer described above is trehalose at a concentration of about 100-300 mM, the concentration of the trehalose described above is preferably about 150-300 mM, and preferably about 200-280 mM, and non-limiting examples of the concentration of the trehalose described above are about 180 mM, 200 mM, 210 mM, 220 mM, 230 mM, 240 mM, 250 mM, 260 mM, 270 mM and 280 mM, preferably 220 mM.

In some embodiments, the stabilizer described above is arginine hydrochloride. In some embodiments, the stabilizer described above is arginine hydrochloride at a concentration of about 30-200 mM, the concentration of the arginine hydrochloride described above is preferably about 50-190 mM, preferably about 100-180 mM, preferably about 120-170 mM, and preferably about 130-150 mM, and non-limiting examples of the concentration of the arginine hydrochloride described above are about 100 mM, 110 mM, 120 mM, 125 mM, 130 mM, 135 mM, 140 mM, 145 mM, 150 mM, 155 mM, 160 mM, 170 mM, 180 mM, 190 mM and 200 mM, preferably 135 mM or 140 mM.

In some embodiments, the stabilizer described above is proline. In some embodiments, the stabilizer described above is proline at a concentration of about 100-300 mM, the concentration of the proline described above is preferably about 150-300 mM, and preferably about 200-280 mM, and non-limiting examples of the concentration of the proline described above are about 180 mM, 200 mM, 210 mM, 220 mM, 230 mM, 240 mM, 250 mM, 260 mM, 270 mM and 280 mM, preferably 240 mM.

In some embodiments, the stabilizer described above is glycine. In some embodiments, the stabilizer described above is glycine at a concentration of about 100-300 mM, the concentration of the glycine described above is preferably about 150-300 mM, and preferably about 200-280 mM, and non-limiting examples of the concentration of the glycine described above are about 180 mM, 200 mM, 210 mM, 220 mM, 230 mM, 240 mM, 250 mM, 260 mM, 270 mM and 280 mM, preferably 260 mM.

In some embodiments, the stabilizer described above is a combination of sodium chloride and mannitol. In some embodiments, the stabilizer described above is a combination of about 30-200 mM of sodium chloride and about 30-200 mM of mannitol, preferably a combination of about 40-150 mM of sodium chloride and about 40-180 mM of mannitol, and preferably a combination of about 40-100 mM of sodium chloride and about 40-150 mM of mannitol. Non-limiting examples of the stabilizer described above are a combination of about 50 mM of sodium chloride and about 120 mM of mannitol, a combination of 50 mM of sodium chloride and about 140 mM of mannitol, and a combination of about 90 mM of sodium chloride and about 50 mM of mannitol.

In some embodiments, the stabilizer described above is a combination of sodium chloride and sucrose. In some embodiments, the stabilizer described above is a combination of about 30-200 mM of sodium chloride and about 30-200 mM of sucrose, preferably a combination of about 40-150 mM of sodium chloride and about 40-180 mM of sucrose, and preferably a combination of about 40-100 mM of sodium chloride and about 40-150 mM of sucrose, non-limiting examples of the stabilizer described above are a combination of about 50 mM of sodium chloride and about 120 mM of sucrose, and a combination of about 90 mM of sodium chloride and about 50 mM of sucrose.

In some embodiments, the stabilizer described above is a combination of arginine hydrochloride and mannitol. In some embodiments, the stabilizer described above is a combination of about 30-200 mM of arginine hydrochloride and about 30-200 mM of mannitol, preferably a combination of about 40-150 mM of arginine hydrochloride and about 40-100 mM of mannitol, and preferably a combination of about 60-120 mM of arginine hydrochloride and about 40-80 mM of mannitol, a non-limiting example of the stabilizer described above is a combination of about 90 mM of arginine hydrochloride and about 50 mM of mannitol.

In some embodiments, the stabilizer described above is a combination of arginine hydrochloride and sucrose. In some embodiments, the stabilizer described above is a combination of about 30-200 mM of arginine hydrochloride and about 30-200 mM of sucrose, preferably a combination of about 40-150 mM of arginine hydrochloride and about 40-100 mM of sucrose, and preferably a combination of about 60-120 mM of arginine hydrochloride and about 40-80 mM of sucrose, a non-limiting example of the stabilizer described above is a combination of about 90 mM of arginine hydrochloride and about 50 mM of sucrose.

In some embodiments, the stabilizer described above is arginine hydrochloride at a concentration of about 120 mM to about 170 mM; or a combination of about 60 mM to about 120 mM of arginine hydrochloride and about 40-80 mM of sucrose; or a combination of about 60 mM to about 120 mM of arginine hydrochloride and about 40-80 mM of mannitol.

In some embodiments, the pharmaceutical composition described above further comprises a surfactant selected from polysorbate 80, polysorbate 20 or poloxamer 188.

In some embodiments, the surfactant described above is selected from polysorbate 80.

In some embodiments, the surfactant described above is selected from polysorbate 20.

In some embodiments, the surfactant described above has a concentration of about 0.001%-0.1%, preferably about 0.01%-0.1%, preferably about 0.02%-0.08%, and more preferably about 0.02%-0.04% %, on w/v basis; As a non-limiting example, the surfactant described above has a concentration of about 0.02%, 0.04% or 0.08%, preferably 0.02%.

In some embodiments, the anti-IL-17A antibody or the antigen-binding fragment thereof described above is selected from murine antibodies, chimeric antibodies and humanized antibodies, preferably humanized antibodies.

In some embodiments, the anti-IL-17A antibody or the antigen-binding fragment thereof described above has a heavy chain variable region as set forth in SEQ ID NO: 19, and a light chain variable region as set forth in SEQ ID NO: 20.

In some embodiments, the anti-IL-17A antibody or the antigen-binding fragment thereof described above has a heavy chain variable region as set forth in SEQ ID NO: 21, and a light chain variable region as set forth in SEQ ID NO: 20.

In some embodiments, the anti-IL-17A antibody or the antigen-binding fragment thereof described above has a heavy chain variable region as set forth in SEQ ID NO: 21, and a light chain variable region as set forth in SEQ ID NO: 22.

In some embodiments, the anti-IL-17A antibody or the antigen-binding fragment thereof described above has a heavy chain variable region as set forth in SEQ ID NO: 23, and a light chain variable region as set forth in SEQ ID NO: 24.

In some embodiments, the anti-IL-17A antibody or the antigen-binding fragment thereof described above has a heavy chain variable region as set forth in SEQ ID NO: 23, and a light chain variable region as set forth in SEQ ID NO: 25.

In some embodiments, the anti-IL-17A antibody or the antigen-binding fragment thereof described above has a heavy chain variable region as set forth in SEQ ID NO: 26, and a light chain variable region as set forth in SEQ ID NO: 27.

In some embodiments, the anti-IL-17A antibody or the antigen-binding fragment thereof described above has a heavy chain amino acid sequence as set forth in SEQ ID NO: 28, and a light chain amino acid sequence as set forth in SEQ ID NO: 29.

In some embodiments, the anti-IL-17A antibody or the antigen-binding fragment thereof described above has a heavy chain amino acid sequence as set forth in SEQ ID NO: 30, and a light chain amino acid sequence as set forth in SEQ ID NO: 29.

In some embodiments, the anti-IL-17A antibody or the antigen-binding fragment thereof described above has a heavy chain amino acid sequence as set forth in SEQ ID NO: 30, and a light chain amino acid sequence as set forth in SEQ ID NO: 31.

In some embodiments, the anti-IL-17A antibody or the antigen-binding fragment thereof described above has a heavy chain amino acid sequence as set forth in SEQ ID NO: 32, and a light chain amino acid sequence as set forth in SEQ ID NO: 33.

In some embodiments, the anti-IL-17A antibody or the antigen-binding fragment thereof described above has a heavy chain amino acid sequence as set forth in SEQ ID NO: 32, and a light chain amino acid sequence as set forth in SEQ ID NO: 34.

In some embodiments, the anti-IL-17A antibody or the antigen-binding fragment thereof described above has a heavy chain amino acid sequence as set forth in SEQ ID NO: 35, and a light chain amino acid sequence as set forth in SEQ ID NO: 36.

In some embodiments, the pharmaceutical composition described above comprises the components shown in any one of the following (1)-(6), wherein the anti-IL-17A antibody or the antigen-binding fragment thereof is as described in any of the embodiments of the present invention :
(1) (a) about 60 mg/mL-180 mg/mL of the anti-IL-17A antibody or the antigen-binding fragment thereof; (b) about 10-30 mM of histidine buffer with a pH of about 5.0-6.0; (c) about 100-300 mM of sorbitol; (d) and about 0.01%-0.1% polysorbate 20 or polysorbate 80; or
(2) (a) about 60 mg/mL-180 mg/mL of the anti-IL-17A antibody or the antigen-binding fragment thereof; (b) about 10-30 mM of histidine buffer with a pH of about 5.0-6.0; (c) about 30 mM to about 200 mM of arginine hydrochloride; (d) and about 0.01%-0.1% polysorbate 20 or polysorbate 80; or
(3) (a) about 60 mg/mL-180 mg/mL of the anti-IL-17A antibody or the antigen-binding fragment thereof; (b) about 10-30 mM of histidine buffer with a pH of about 5.0-6.0; (c) a combination of about 30 mM to about 200 mM of sodium chloride and about 30-200 mM of mannitol; (d) and about 0.01%-0.1% polysorbate 20 or polysorbate 80; or
(4) (a) about 60 mg/mL-180 mg/mL of the anti-IL-17A antibody or the antigen-binding fragment thereof; (b) about 10-30 mM of histidine buffer with a pH of about 5.0-6.0; (c) a combination of about 30 mM to about 200 mM of sodium chloride and about 30-200 mM of sucrose; (d) and about 0.01%-0.1% polysorbate 20 or polysorbate 80; or
(5) (a) about 60 mg/mL-180 mg/mL of the anti-IL-17A antibody or the antigen-binding fragment thereof; (b) about 10-30 mM of histidine buffer with a pH of about 5.0-6.0; (c) a combination of about 30 mM to about 200 mM of arginine hydrochloride and about 30-200 mM of mannitol; (d) and about 0.01%-0.1% polysorbate 20 or polysorbate 80; or (6) (a) about 60 mg/mL-180 mg/mL of the anti-IL-17A antibody or the antigen-binding fragment thereof; (b) about 10-30 mM of histidine buffer with a pH of about 5.0-6.0; (c) a combination of about 30 mM to about 200 mM of arginine hydrochloride and about 30-200 mM of sucrose; (d) and about 0.01%-0.1% polysorbate 20 or polysorbate 80.

In some embodiments, the pharmaceutical composition described above comprises the components shown in any one of the following (7)-(12):
(7) (a) about 80 mg/mL of the anti-IL-17A antibody or the antigen-binding fragment thereof; (b) about 20 mM of histidine buffer with a pH of about 5.5; (c) a combination of about 50 mM of sodium chloride and about 140 mM of mannitol; (d) and about 0.02% polysorbate 20; or
(8) (a) about 150 mg/mL of the anti-IL-17A antibody or the antigen-binding fragment thereof; (b) about 30 mM of histidine buffer with a pH of about 5.5; (c) about 135 mM of arginine hydrochloride; (d) and about 0.02% polysorbate 20; or
(9) (a) about 150 mg/mL of the anti-IL-17A antibody or the antigen-binding fragment thereof; (b) about 30 mM of histidine buffer with a pH of about 5.5; (c) a combination of about 50 mM of sodium chloride and about 120 mM of sucrose; (d) and about 0.02% polysorbate 20;
(10) (a) about 150 mg/mL of the anti-IL-17A antibody or the antigen-binding fragment thereof; (b) about 30 mM of histidine buffer with a pH of about 5.5; (c) a combination of about 90 mM of arginine hydrochloride and about 50 mM of mannitol; (d) and about 0.02% polysorbate 20;
(11) (a) about 150 mg/mL of the anti-IL-17A antibody or the antigen-binding fragment thereof; (b) about 30 mM of histidine buffer with a pH of about 5.5; (c) a combination of about 90 mM of arginine hydrochloride and about 50 mM of sucrose; (d) and about 0.02% polysorbate 20;
(12) (a) about 150 mg/mL of the anti-IL-17A antibody or the antigen-binding fragment thereof; (b) about 15 mM of histidine buffer with a pH of about 5.5; (c) a combination of about 90 mM of arginine hydrochloride and about 50 mM of sucrose; (d) and about 0.02% polysorbate 20.

In some embodiments, the pharmaceutical composition described above comprises the components shown in any one of the following (i)-(vi), wherein the anti-IL-17A antibody or the antigen-binding fragment thereof is as described in any of the embodiments of the present invention :
(i) (a) about 80 mg/mL to 150 mg/mL of the anti-IL-17A antibody or the antigen-binding fragment thereof; (b) about 10-30 mM of histidine buffer with a pH of about 5.5-6.0; (c) about 200-280 mM of sorbitol; (d) and about 0.02%-0.04% polysorbate 20 or polysorbate 80; or
(ii) (a) about 80 mg/mL to 150 mg/mL of the anti-IL-17A antibody or the antigen-binding fragment thereof; (b) about 10-30 mM of histidine buffer with a pH of about 5.5-6.0; (c) about 120 mM to about 170 mM of arginine hydrochloride; (d) and about 0.02%-0.04% polysorbate 20 or polysorbate 80; or
(iii) (a) about 80 mg/mL to 150 mg/mL of the anti-IL-17A antibody or the antigen-binding fragment thereof; (b) about 10-30 mM of histidine buffer with a pH of about 5.5-6.0; (c) a combination of about 40 mM to about 100 mM of sodium chloride and about 40-150 mM of mannitol; (d) and about 0.02%-0.04% polysorbate 20 or polysorbate 80; or
(iv) (a) about 80 mg/mL to 150 mg/mL of the anti-IL-17A antibody or the antigen-binding fragment thereof; (b) about 10-30 mM of histidine buffer with a pH of about 5.5-6.0; (c) a combination of about 40 mM to about 100 mM of sodium chloride and about 40-150 mM of sucrose; (d) and about 0.02%-0.04% polysorbate 20 or polysorbate 80; or
(v) (a) about 80 mg/mL to 150 mg/mL of the anti-IL-17A antibody or the antigen-binding fragment thereof; (b) about 10-30 mM of histidine buffer with a pH of about 5.5-6.0; (c) a combination of about 60 mM to about 120 mM of arginine hydrochloride and about 40-80 mM of mannitol; (d) and about 0.02%-0.04% polysorbate 20 or polysorbate 80; or
(vi) (a) about 80 mg/mL to 150 mg/mL of the anti-IL-17A antibody or the antigen-binding fragment thereof; (b) about 10-30 mM of histidine buffer with a pH of about 5.5-6.0; (c) a combination of about 60 mM to about 120 mM of arginine hydrochloride and about 40-80 mM of sucrose; (d) and about 0.02%-0.04% polysorbate 20 or polysorbate 80.

In some embodiments, the pharmaceutical composition is a liquid formulation or a lyophilized formulation.

In some embodiments, the pharmaceutical composition is a liquid formulation.

In some embodiments, the liquid formulation or the lyophilized formulation described above is stable at 2-8°C for at least 3 months, at least 6 months, at least 12 months, at least 18 months or at least 24 months.

In some embodiments, the liquid formulation or the lyophilized formulation described above is stable at 40°C for at least 7 days, at least 14 days or at least 28 days.

The present invention also provides the use of the pharmaceutical composition described above in the preparation of a medicament for the treatment or prevention of IL-17A-mediated diseases.

In some embodiments, the diseases described above include inflammatory diseases and autoimmune diseases, such as arthritis, rheumatoid arthritis, ankylosing spondylitis, chronic obstructive pulmonary disease, systemic lupus erythematosus (SLE), lupus nephritis, asthma, multiple sclerosis, cystic fibrosis and psoriasis.

### Brief Description of the Drawings

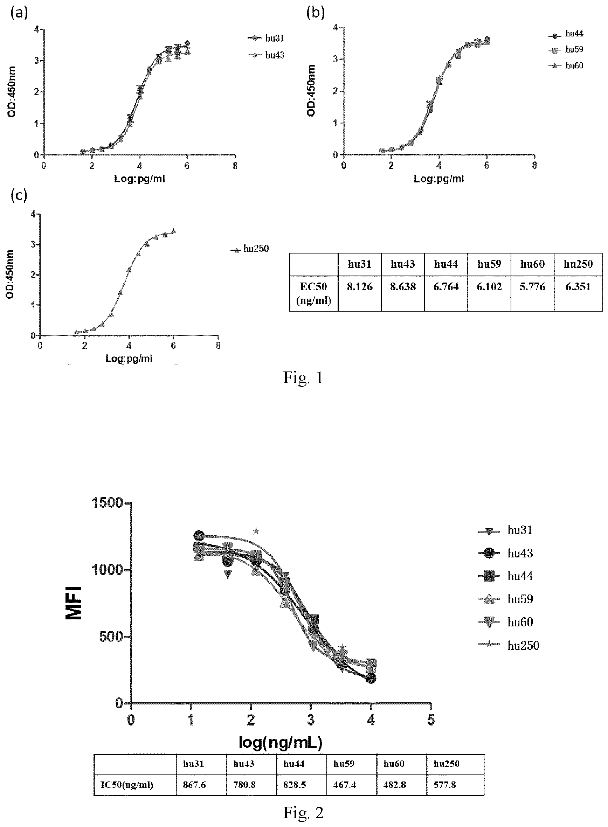

Fig. 1: binding of humanized antibodies to human IL-17A assayed by ELISA. Among the humanized antibodies, the antibodies hu31, hu43, hu44, hu59, hu60 and hu250 have a high specificity for binding to human IL-17A, and the EC50s thereof are 8.13 ng/mL, 8.64 ng/mL, 6.764 ng/mL, 6.102 ng/mL, 5.776 ng/mL and 6.351 ng/mL, respectively.
Fig. 2: effect of humanized antibodies for blocking the binding of human IL-17A to IL-17RA on 293F cells assayed by FACS. Among the humanized antibodies, the antibodies hu31, hu43, hu44, hu59, hu60 and hu250 have high blocking effect, and their IC50s are 867.6 ng/mL, 780.8 ng/mL, 828.5 ng/mL, 467.4 ng/mL, 482.8 ng/mL and 577.8 ng/mL, respectively.

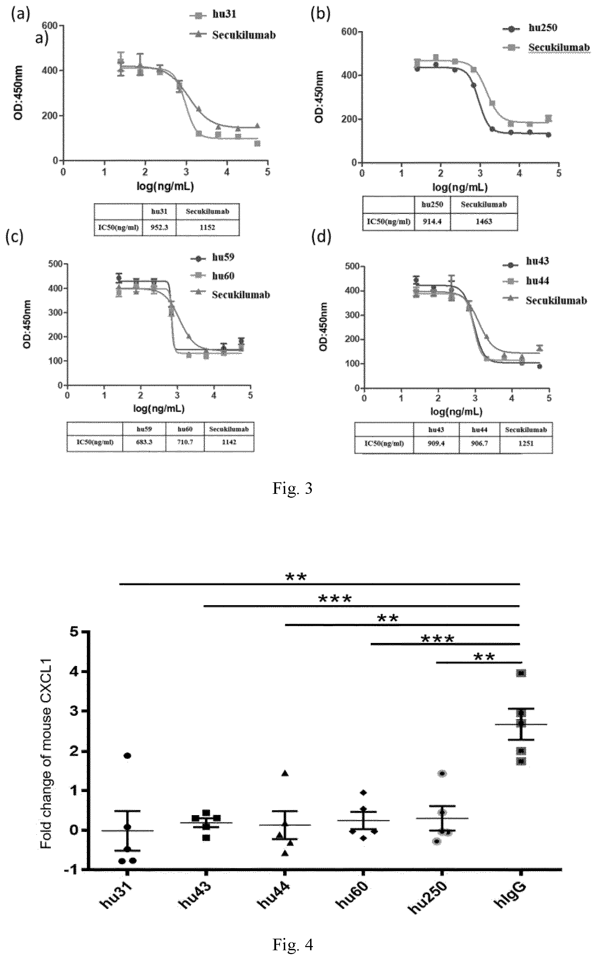

Fig. 3: effect of humanized antibodies for blocking IL-17A-mediated secretion of CXCL1 by epithelial cells assayed by ELISA. The antibodies hu31, hu43, hu44, hu59, hu60 and hu250 all efficiently inhibited the IL-17A-induced expression of CXCL1 by epithelial cells, and the blocking effect is stronger than that of a control antibody.
Fig. 4: effect of humanized antibodies for blocking IL-17A-mediated biological activity *in vivo* assayed by ELISA. The antibodies hu31, hu43, hu44, hu60 and hu250 efficiently inhibited the IL-17A-induced expression of CXCL1 in mice, and the blocking effect is stronger than that of a control antibody.

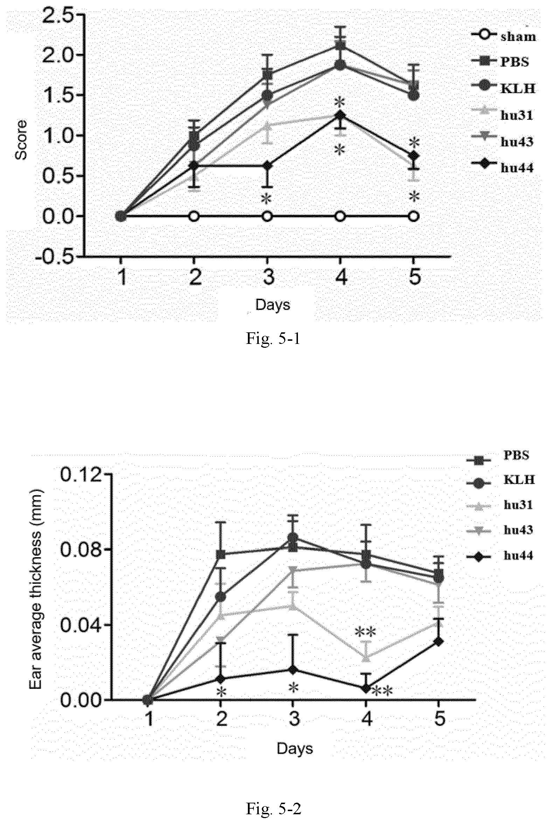

Fig. 5-1: effect of humanized antibody administration on clinical scores in mice with imiquimod-induced psoriasis. The dministration of hu31 and hu44 could significantly inhibit the scaling, induration, and red and swollen of the skin of the mouse model with imiquimod-induced psoriasis, that is, the clinical scores decrease (*P < 0.05 vs KLH).
Fig. 5-2: effect of humanized antibodies on the degree of ear swelling in mice with imiquimod-induced psoriasis. Administration of hu31 and hu44 can significantly improve the degree of ear swelling. (*P < 0.05 vs KLH, **P < 0.01 vs KLH.).

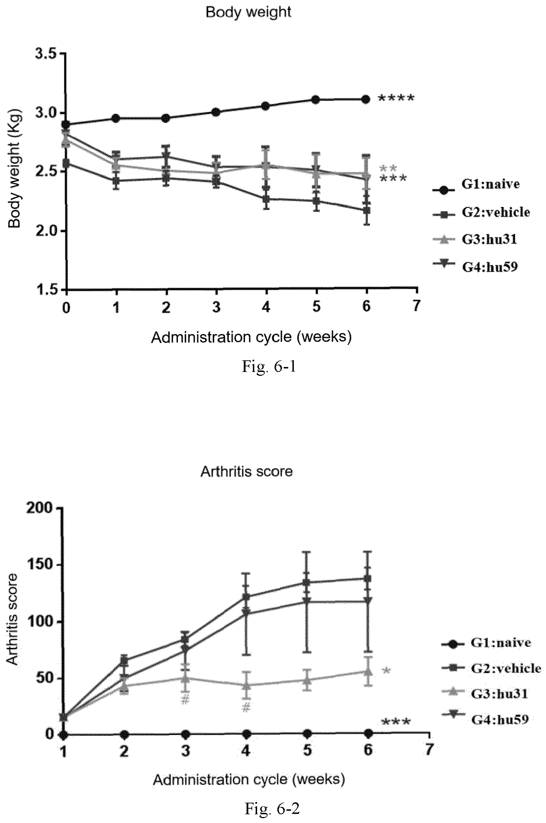

Fig. 6-1: effect of humanized antibodies on collagen type II-induced body weight in female cynomolgus monkeys. hu31 and hu59 have a certain improvement effect on weight loss caused by arthritis (**P < 0.01, ^{∗∗∗∗}P < 0.0001, compared with "G2: vehicle group"; One-way ANOVA/Dunnett).
Fig. 6-2: effect of humanized antibodies on collagen type II-induced arthritis scores in female cynomolgus monkeys. hu31 significantly inhibited the increasing trend of the arthritis clinical scores in cynomolgus monkeys (***P < 0.001, #P < 0.05, compared with G2: vehicle group; One-way ANOVA/Dunnett).

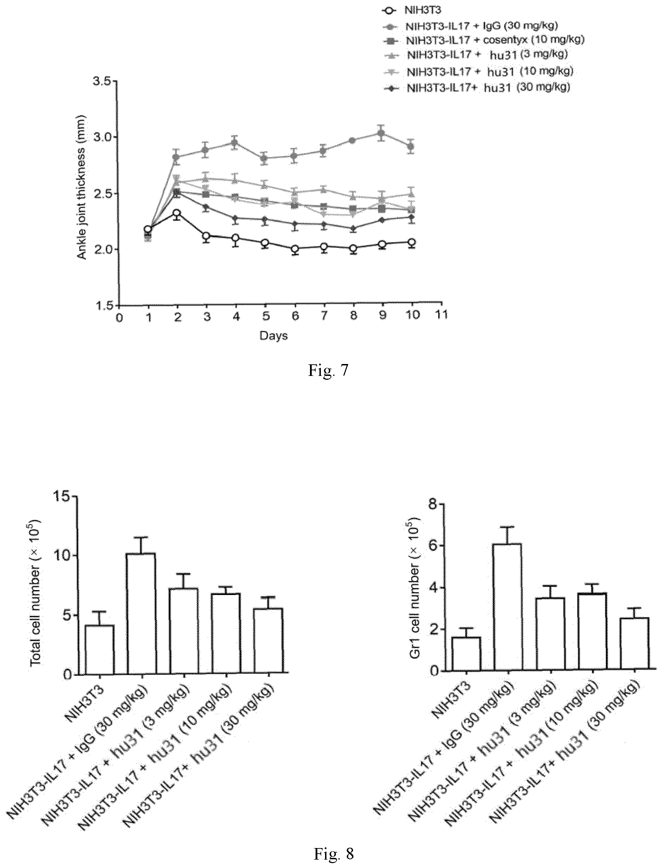

Fig. 7: effect of humanized antibodies on NIH3T3-IL17 cell-induced joint swelling in mice.
Fig. 8: effect of humanized antibodies on NIH3T3-IL17 cell-induced air sac inflammation in mice.

### Detailed Description of Embodiments

### Definition and Description

In order that the present invention may be more readily understood, certain technical and scientific terms are specifically defined below. Unless explicitly defined otherwise herein, all other technical and scientific terms used herein have the same meaning as commonly understood by one of ordinary skill in the art to which this invention pertains. It is to be understood that this invention is not limited to particular methods, reagents, compounds, compositions or biological systems, which can, of course, vary. It is also to be understood that the terminology used herein is for the purpose of describing particular embodiments only and is not intended to be limiting.

Unless the content is clearly stated otherwise, the singular forms "a", "an" and "the" used in this specification and the appended claims include plural referents. Thus, for example, reference to "a polypeptide" includes a combination of two or more polypeptides.

The term "pharmaceutical composition" or "formulation" refers to a mixture comprising one or more of the antibodies described herein and other components, such as a physiologically and pharmaceutically acceptable carrier and an excipient. The purpose of a pharmaceutical composition is to promote the administration to an organism, which will facilitate the absorption of active ingredients and thus exert biological activities.

The term "liquid formulation" refers to a formulation in a liquid state and is not intended to refer to a resuspended lyophilized formulation. The liquid formulation of the present invention is stable on storage, and its stability is independent of lyophilization (or other state-changing methods, such as spray drying).

The term "aqueous liquid formulation" refers to a liquid formulation using water as a solvent. In some embodiments, the aqueous liquid formulation is a formulation that do not require lyophilization, spray drying, and/or freezing to maintain stability (for example, chemical and/or physical stability and/or biological activity).

The term "excipient" refers to a reagent that can be added to a formulation to provide desired properties (for example, consistency, improved stability) and/or to adjust osmotic pressure. Examples of common excipients include, but are not limited to, sugars, polyols, amino acids, surfactants, and polymers.

As used herein, "about" when referring to a measurable value (for example, amount, duration, *etc.*) is intended to encompass a variation of ± 20% or ± 10% from the specified value, including ± 5%, ± 1% and ± 0.1 % as these variations are suitable for carrying out the disclosed method.

The term "buffer with a pH of about 5.0-6.5" refers to a reagent which renders a solution containing the reagent resistant to pH changes through the action of its acid/base conjugate component. The buffer used in the formulation of the present invention may have a pH in the range of about 5.0 to about 6.5, or a pH in the range of about 5.5 to about 6.5, or a pH in the range of about 5.0 to about 6.0.

Herein, examples of "buffer" that controls pH within this range include acetate (for example, sodium acetate), succinate (for example, sodium succinate), gluconic acid, histidine, histidine hydrochloride, methionine, citrate, phosphate, citrate/phosphate, imidazole, ethanoic acid, ethanoate, citrate and combinations thereof, and other organic acid buffers.

A "histidine buffer" is a buffer containing histidine ions. Examples of histidine buffer include histidine and histidine salts, such as histidine hydrochloride, histidine acetate, histidine phosphate, histidine sulfate, and the like, such as a histidine buffer containing histidine and histidine hydrochloride; The histidine buffer of the present invention also includes a histidine buffer containing histidine and acetate (for example, sodium acetate or potassium acetate).

A "citrate buffer" is a buffer containing citrate ions. Examples of citrate buffer include citric acid-sodium citrate, citric acid-potassium citrate, citric acid-calcium citrate, citric acid-magnesium citrate, and the like. A preferred citrate buffer is citric acid-sodium citrate buffer.

A "acetate buffer" is a buffer containing acetate ions. Examples of acetate buffer include acetic acid-sodium acetate, acetic acid-potassium acetate, acetic acid-calcium acetate, acetic acid-magnesium acetate, and the like. The preferred acetate buffer is acetic acid-sodium acetate buffer.

A "succinate buffer" is a buffer containing succinate ions. Examples of succinate buffer include succinic acid-sodium succinate, succinic acid-potassium succinate, succinic acid-calcium succinate, succinic acid-magnesium succinate, and the like. The preferred succinate buffer is succinic acid-sodium succinate buffer.

The term "stabilizer" means a pharmaceutically acceptable excipient which protects active pharmaceutical ingredients and/or formulations from chemical and/or physical degradation during manufacture, storage and application. The stabilizer includes, but are not limited to, sugars, amino acids, salts, polyols and their metabolites as defined below, for example, sodium chloride, calcium chloride, magnesium chloride, mannitol, sorbitol, sucrose, trehalose, arginine or its salts (such as arginine hydrochloride), glycine, alanine (alpha-alanine, beta-alanine), betaine, leucine, lysine, glutamic acid, aspartic acid, proline, 4-hydroxyproline, sarcosine, gamma-aminobutyric acid (GABA), opines, alanopine, octopine, strombine and trimethylamine N-oxide (TMAO), human serum albumin (hsa), bovine serum albumin (bsa), alpha-casein, globulin, alpha-lactalbumin, LDH, lysozyme, myoglobin, ovalbumin, and RNAaseA. Some stabilizers, such as sodium chloride, calcium chloride, magnesium chloride, mannitol, sorbitol, sucrose, *etc.,* can also play a role in controlling osmotic pressure. The stabilizer specifically used in the present invention is selected from one or more of polyols, amino acids, salts, and sugars. The preferred salt is sodium chloride, the preferred sugars are sucrose and trehalose, and the preferred polyols are sorbitol and mannitol. The preferred amino acids are arginine or a salt thereof (for example, arginine hydrochloride), glycine and proline. Preferred stabilizer is sodium chloride, mannitol, sorbitol, sucrose, trehalose, arginine hydrochloride, glycine, proline, sodium chloride-sorbitol, sodium chloride-mannitol, sodium chloride-sucrose, sodium chloride-trehalose, arginine hydrochloride-mannitol, arginine hydrochloride-sucrose, more preferably arginine hydrochloride, sodium chloride-sucrose, arginine hydrochloride-mannitol, arginine hydrochloride- sucrose, more preferably arginine hydrochloride-sucrose.

The term "viscosity" as used herein may be "kinematic viscosity" or "absolute viscosity". "Kinematic viscosity" is a measure of the resistance of a fluid to flow under the influence of gravity. "Absolute viscosity", sometimes called dynamic viscosity or simply viscosity, is the product of kinematic viscosity and fluid density (absolute viscosity = kinematic viscosity X density). The dimension of kinematic viscosity is L2/T, where L is length and T is time. Typically, kinematic viscosity is expressed in centistokes (cSt). The unit of international system of units for kinematic viscosity is mm²/s, *i.e.,* 1cSt. Absolute viscosity is expressed in centipoise (cP) units. The unit of international system of units for absolute viscosity is milliPascal per second (mPa-s), where 1 cP = 1 mPa·s.

For the pharmaceutical composition of the present invention, the term "low level viscosity" as used herein refers to an absolute viscosity of less than about 15 centipoise (cP). For example, when measured using standard viscosity measurement techniques, if the formulation exhibits an absolute viscosity of about 15 cP, about 14 cP, about 13 cP, about 12 cP, about 11 cP, about 10 cP, about 9 cP, about 8 cP, about 7 cP, about 6 cP, about 5 cP, about 4 cP, about 3 cP, about 2 cP, about 1 cP, or less, then the pharmaceutical composition of the present invention would be considered to have "low viscosity". For the pharmaceutical composition of the present invention, the term "medium viscosity" as used herein refers to an absolute viscosity between about 35 cP and about 15 cP. For example, when measured using standard viscosity measurement techniques, if the formulation exhibits an absolute viscosity of about 34 cP, about 33 cP, about 32 cP, about 31 cP, about 30 cP, about 29 cP, about 28 cP, about 27 cP, about 26 cP, about 25 cP, about 24 cP, about 23 cP, about 22 cP, about 21 cP, about 20 cP, about 19 cP, 18 cP, about 17 cP, about 16 cP, or about 15.1 cP, then the pharmaceutical composition of the present invention would be considered to have a "medium viscosity". The pharmaceutical composition of the present invention may, in certain embodiments, exhibit a low level of viscosity. In some embodiments, it is found by comparing the viscosity of different excipients that the viscosity level obtained using arginine or a salt thereof is substantially lower than that obtained using other excipients. In some embodiments, the viscosity level of the histidine buffer system is lower than that of other buffer systems by comparing different buffer systems.

The term "surfactant" generally includes a reagent that protects proteins, such as antibodies, from air/solution interface induced stress, solution/surface induced stress to reduce aggregation of antibodies or to minimize the formation of particulate matter in the formulation. Exemplary surfactants include, but are not limited to, nonionic surfactants such as polyoxyethylene sorbitan fatty acid esters (for example, polysorbate 20 and polysorbate 80), polyethylene-polypropylene copolymers, polyethylene-polypropylene glycol, polyoxyethylene-stearate, polyoxyethylene alkyl ethers (such as polyoxyethylene monolauryl ether), alkyl phenyl polyoxyethylene ether (Triton-X), polyoxyethylene-polyoxypropylene copolymers (poloxamer, Pluronic) and sodium dodecyl sulfate (SDS).

The term "isotonicity" means that the formulation has substantially the same osmotic pressure as human blood. An isotonic formulation generally has an osmolarity of about 250 to 350 mOsm. Isotonicity can be measured using a vapor pressure or freezing point depression osmometer.

The term "stable" formulation is a formulation in which an antibody substantially retains its physical and/or chemical stability and/or biological activity during the manufacturing process and/or storage. A pharmaceutical formulation can be stable even if the contained antibody fails to retain 100% of its chemical structure or biological function after a certain period of storage. In some cases, an antibody that retains about 90%, about 95%, about 96%, about 97%, about 98%, or about 99% of its structure or function after a certain period of storage may also be considered "stable". Various analytical techniques for measuring protein stability are available in the art and are reviewed in Peptide and Protein Drug Delivery 247-301, edited by Vincent Lee, Marcel Dekker, Inc ., New York, N.Y., Pubs. (1991), and Jones, A. (1993) Adv. Drug Delivery Rev. 10: 29-90 (both incorporated by reference).

The stability of a formulation can be measured by, among other methods, determining the percentage of native antibodies remaining in the formulation after storage at a given temperature for a given period of time. Among other methods, the percentage of native antibodies can be measured by size exclusion chromatography (for example, Size Exclusion High Performance Liquid Chromatography [SEC-HPLC]), "native" means unaggregated and undegraded. In some embodiments, protein stability is determined as the percentage of monomeric proteins in solution with a low percentage of degraded (for example, fragmented) and/or aggregated proteins. In some embodiments, the formulation is stable for storage at room temperature, about 25-30°C or 40°C for at least 2 weeks, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months or longer, with no more than about 6%, 5%, 4%, 3%, 2%, 1%, 0.5%, or 0.1% of the antibodies in aggregated form.

Stability can be measured by, among other methods, determining the percentage of antibodies ("acidic form") migrating in the more acidic fraction of this antibody main fraction ("main charge form") during ion exchange, where the stability is inversely proportional to that percentage of antibodies in acidic form. Among other methods, the percentage of "acidified" antibodies can be measured by ion exchange chromatography (for example, cation exchange high performance liquid chromatography [CEX-HPLC]). In some embodiments, an acceptable degree of stability means that upon storage of the formulation at a certain temperature for a certain period of time, the detectable antibodies in acidic form are no more than about 49%, 45%, 40%, 35%, 30%, 25%, 20%, 15%, 10%, 5%, 4%, 3%, 2%, 1%, 0.5%, or 0.1%. The certain period of time of storage prior to measuring stability can be at least 2 weeks, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months or longer. When assessing stability, a certain temperature that allows storage of a pharmaceutical formulation can be any temperature in the range of about -80°C to about 45°C, for example, about -80°C, about -30°C, about -20°C C, about 0°C, about 2-8°C, about 5°C, about 25°C or about 40°C.

If the antibodies show substantially no evidence of, for example, aggregation, precipitation and/or denaturation upon visual inspection of color and/or clarity or as measured by UV light scattering or by size exclusion chromatography, then the antibodies "retain their physical stability" in that pharmaceutical composition. Aggregation is the process by which individual molecules or complexes associate covalently or non-covalently to form aggregates. Aggregation can proceed to the degree where a visible precipitate is formed.

The stability of the formulation, for example, physical stability, can be assessed by methods well known in the art, including measuring the apparent extinction (absorbance or optical density) of the sample. Such a extinction measurement correlates with the turbidity of the formulation. The turbidity of the formulation is, in part, an inherent property of proteins dissolved in a solution, and is typically measured by turbidimetry, and is expressed in Nephelometric Turbidity Unit (NTU).

The level of turbidity that varies, for example, with the concentration of one or more components (for example, protein and/or salt concentration) in solution is also referred to as the "opacification" or "opacification appearance" of the formulation. Turbidity level can be calculated with reference to a standard curve generated using a suspension having known turbidity. Reference standards for determining the turbidity level of the pharmaceutical composition can be based on the European Pharmacopoeia standard (European Pharmacopoeia, 4th edition, "Directorate for the Quality of Medicine of the Council of Europe" (EDQM), Strasbourg, France). According to the European Pharmacopoeia standard, a clear solution is defined as a solution with a turbidity lower than or equal to a reference suspension turbidity of about 3 according to the European Pharmacopoeia standard. Turbidity measurement in turbidimetry can detect Rayleigh scattering in the absence of association or non-ideal effects, Rayleigh scattering typically vary linearly with concentration. Other methods for assessing physical stability are known in the art.

An antibody "retains its chemical stability" in a pharmaceutical composition if its chemical stability at a given time point is such that the antibody is considered to still retain its biological activity as defined hereinafter. Chemical stability can be assessed by, for example, detecting or quantifying chemically changed forms of the antibody. Chemical changes can include size changes (for example, clipping), which can be assessed using, for example, size exclusion chromatography, SDS-PAGE, and/or matrix-assisted laser desorption ionization/time-of-flight mass spectrometry (MALDI/TOF MS). Other types of chemical changes include charge changes (for example, resulting form deamidation or oxidation), which can be assessed by, for example, ion exchange chromatography.

An antibody "retains its biological activity" in a pharmaceutical composition if the antibody in the pharmaceutical composition is biologically active for its intended purpose. For example, if after the formulation is stored at a temperature such as 5°C, 25°C, 45°C, *etc.* for a certain period of time (for example, 1 to 12 months), the affinity of the anti-IL-17A antibody contained in the formulation for binding to IL-17A is at least 90%, 95% or more of the binding affinity of the antibody prior to the storage, then the formulation of the present invention is considered to be stable. Binding affinity can also be determined using techniques such as ELISA or plasmon resonance.

In the context of the present invention, a "therapeutically effective amount" or "effective amount" of an antibody in the pharmacological sense refers to an amount effective in the prevention or treatment or alleviation of the symptoms of the disorder that the antibody can effectively treat. In the present invention, a "therapeutically effective amount" or "therapeutically effective dose" of a drug is any amount of a drug that protects a subject from the onset of a disease or promotes the regression of a disease when used alone or in combination with another therapeutic agent. The regression of the disease is evidenced by the reduction in the severity of the symptoms of the disease, the increase in the frequency and duration of the asymptomatic period of the disease, or the prevention of injury or disability caused by the pain of the disease. The ability of a drug to promote disease regression can be evaluated using a variety of methods known to those skilled in the art, such as determining the activity of the agent in human subjects during clinical trials, in animal model systems that predict the efficacy in human, or by *in vitro* assays. A therapeutically effective amount of a drug includes a "prophylactically effective amount", that is, any amount of a drug that inhibits the development or recurrence of a disease when administered to a subject at risk of developing the disease or a subject suffering from recurrence of the disease, alone or in combination with other therapeutic drugs.

The terms "subject" or "patient" are intended to include mammalian organisms. Examples of subject/patient include human and non-human mammals, such as non-human primates, dogs, cows, horses, pigs, sheep, goats, cats, mice, rabbits, rats and transgenic non-human animals. In a specific embodiment of the invention, the subject is human.

The terms "administrate", "dose" and "treat" refer to the introduction of a composition containing a therapeutic agent into a subject using any of various methods or delivery systems known to those skilled in the art. The administration route of an anti-PD-1 antibody includes intravenous, intramuscular, subcutaneous, peritoneal, spinal or other parenteral administration routes, such as injection or infusion. "Parenteral administration" refers to administration routes usually by injection other than enteral or local administration, including but not limited to intravenous, intramuscular, intraarterial, intrathecal, intralymphatic, intralesional, intracapsular, intraorbital, intracardiac, intradermal, intraperitoneal, transtracheal, subcutaneous, subepidermal, intraarticular, subcapsular, subarachnoid, intraspinal, intradural and intrasternal injection and infusion, and electroporation *in vivo.*

### Anti-IL-17A antibody

The term "antibody" as used herein should be understood to include an intact antibody molecule and an antigen-binding fragment thereof. The term "antigen-binding portion" or "antigen-binding fragment" (or simply "antibody portion" or "antibody fragment") of an antibody as used herein refers to one or more fragments in the antibody that retain the ability to specifically bind to human IL-17A (interleukin 17A) or an epitope thereof.

The term "full-length antibody" or "intact antibody molecule" as used herein refers to an immunoglobulin molecule comprising four peptide chains, two heavy (H) chains (about 50-70 kDa at full length) and two light (L) chains (about 25 kDa in full length) connected to each other by disulfide bonds. Each heavy chain consists of a heavy chain variable region (abbreviated herein as VH) and a heavy chain constant region (abbreviated herein as CH). The heavy chain constant region consists of three domains, CH1, CH2 and CH3. Each light chain consists of a light chain variable region (abbreviated herein as VL) and a light chain constant region. The light chain constant region consists of one domain CL. The VH and VL regions can be further subdivided into highly variable complementarity determining regions (CDRs) and more conserved regions called framework regions (FRs) spaced by CDRs. Each VH and VL region consists of 3 CDRs and 4 FRs arranged in the following order: FR1, CDR1, FR2, CDR2, FR3, CDR3, and FR4 from amino terminus to carboxy terminus. The variable regions of the heavy and light chains contain binding domains that interact with antigens. The constant region of the antibody can mediate the binding of the immunoglobulin to a host tissue or factor (comprising various cells (e.g., effector cells) of the immune system and the first component of the classical complement system (Clq)).

As used herein, the term "CDR" refers to a complementarity determining region within the variable sequence of an antibody. There are 3 CDRs in each variable region of the heavy and light chains respectively, and the CDRs are designated HCDR1, HCDR2 and HCDR3 or LCDR1, LCDR2 and LCDR3 for each heavy and light chain variable region. The exact boundaries of these CDRs are defined differently according to different systems. The system described by Kabat (supra) provides not only an unambiguous residue numbering system applicable to any variable region of an antibody, but also exact residue boundaries that define the 3 CDRs. These CDRs can be called Kabat CDRs. Chothia *et al* found that some subparts in Kabat CDR adopt almost the same peptide skeleton configuration, despite the large diversity at the amino acid sequence level (Chothia et al, (1987) Mol. Biol. 196:901-917; Chothia et al, (1989) Nature 342:877-883). Other boundaries defining CDRs that overlap with the Kabat CDRs have been described by Padlan (1995) FASEB J. 9:133-139 and MacCallum (1996) J. Mol. Biol. 262(5):732-45. Other definitions for CDR boundaries may not strictly follow one of the systems described herein, although these CDR boundaries may be shortened or lengthened, they will still overlap with the Kabat CDRs, this is due to the predicted or experimental finding that specific residues or sets of residues or even whole CDR do not significantly affect antigen binding. The method used herein can utilize CDRs defined according to any of these systems, although certain embodiment uses CDRs defined by Kabat or Chothia.

As used herein, "antigen-binding fragment" includes a fragment of an antibody or a derivative thereof, typically including at least a fragment of the antigen-binding region or the variable region (for example, one or more CDRs) of the parent antibody, and the fragment of the antibody or the derivative thereof retains at least some of the binding specificity of the parent antibody. Examples of antigen-binding fragment include, but are not limited to, Fab, Fab', F(ab')2 and Fv fragments; diabodies; linear antibodies; single-stranded antibody molecules, such as sc-Fv; and nanobodies and multispecific antibodies formed from antibody fragments. When the binding activity of an antibody is expressed on a molar concentration basis, a binding fragment or a derivative thereof typically retains at least 10% of the antigen binding activity of the parent antibody. Preferably the binding fragment or the derivative thereof retains at least 20%, 50%, 70%, 80%, 90%, 95% or 100% or more of the antigen binding affinity of the parent antibody. It is also contemplated that an antigen-binding fragment of an antibody may include conservative or non-conservative amino acid substitutions that do not significantly change its biological activity (referred to as "conservative variants" or "functionally conservative variants" of the antibody).

The anti-IL-17A antibody or the antigen-binding fragment thereof of the present invention include any one of the anti-IL-17A antibodies described in the application number PCT/CN 2019/124486, the entire contents of which are incorporated herein by reference. In some embodiments, the CDR sequences of the antibodies used in the methods and compositions of the invention include the CDR sequences from the antibody hu31 described in PCT/CN 2019/124486.

The non-limiting, exemplary antibodies used in the examples herein are selected from the humanized antibodies hu31, hu43, hu44, hu59, hu60 and hu250 described in CN 201811515045.7, the variable regions and CDR amino acid sequences of these humanized antibodies are shown in Table 1, and the full-length amino acid sequences of the light/heavy chains of these humanized antibodies are shown in Table 2.

**Table 1: Variable regions and CDR sequences of humanized antibody**

| Antibody | hu31 | hu43 | hu44 | hu59 | hu60 | hu250 |
|---|---|---|---|---|---|---|
| HCDR1 | SEQ ID NO: 1 | SEQ ID NO: 1 | SEQ ID NO: 1 | SEQ ID NO: 7 | SEQ ID NO: 7 | SEQ ID NO: 13 |
| HCDR2 | SEQ ID NO: 2 | SEQ ID NO: 2 | SEQ ID NO: 2 | SEQ ID NO: 8 | SEQ ID NO: 8 | SEQ ID NO: 14 |
| HCDR3 | SEQ ID NO: 3 | SEQ ID NO: 3 | SEQ ID NO: 3 | SEQ ID NO: 9 | SEQ ID NO: 9 | SEQ ID NO: 15 |
| LCDR1 | SEQ ID NO: 4 | SEQ ID NO: 4 | SEQ ID NO: 4 | SEQ ID NO: 10 | SEQ ID NO: 10 | SEQ ID NO: 16 |
| LCDR2 | SEQ ID NO: 5 | SEQ ID NO: 5 | SEQ ID NO: 5 | SEQ ID NO: 11 | SEQ ID NO: 11 | SEQ ID NO: 17 |
| LCDR3 | SEQ ID NO: 6 | SEQ ID NO: 6 | SEQ ID NO: 6 | SEQ ID NO: 12 | SEQ ID NO: 12 | SEQ ID NO: 18 |
| VH | SEQ ID NO: 19 | SEQ ID NO: 21 | SEQ ID NO: 21 | SEQ ID NO: 23 | SEQ ID NO: 23 | SEQ ID NO: 26 |
| VL | SEQ ID NO: 20 | SEQ ID NO: 20 | SEQ ID NO: 22 | SEQ ID NO: 24 | SEQ ID NO: 25 | SEQ ID NO: 27 |

**Table 2: Full-length amino acid sequences of humanized antibody**

| Humanized antibody | Heavy chain (HC) | Light chain (LC) |
|---|---|---|
| hu31 | SEQ ID NO: 28 | SEQ ID NO: 29 |
| hu43 | SEQ ID NO: 30 | SEQ ID NO: 29 |
| hu44 | SEQ ID NO: 30 | SEQ ID NO: 31 |
| hu59 | SEQ ID NO: 32 | SEQ ID NO: 33 |
| hu60 | SEQ ID NO: 32 | SEQ ID NO: 34 |
| hu250 | SEQ ID NO: 35 | SEQ ID NO: 36 |

### Pharmaceutical formulation

The pharmaceutical composition of the present invention is a highly stable pharmaceutical composition comprising a humanized antibody that specifically binds to IL-17A. The present invention finds that the combination of arginine salt (such as arginine hydrochloride) and sucrose can obviously improve the stability of the pharmaceutical composition.

The pharmaceutical composition of the present invention is a liquid formulation containing a high concentration of active antibodies and having high stability and low viscosity. In particular, the present invention finds that a formulation containing arginine salt has significantly lower viscosity than formulations containing only other excipients. In addition, the viscosity level obtained by using the histidine buffer system is significantly lower than that obtained by using other buffer systems.

The present invention provides a pharmaceutical composition, comprising: (1) a buffer; (2) an anti-IL-17A antibody or an antigen-binding fragment thereof.

The antibody in the pharmaceutical composition of the present invention can be a murine antibody, a chimeric antibody, a humanized antibody, preferably a humanized antibody, and can have HCDR1, HCDR2 and HCDR3 as shown in SEQ ID NO: 1, SEQ ID NO: 2 and SEQ ID NO: 3, respectively, and LCDR1, LCDR2 and LCDR3 as shown in SEQ ID NO: 4, SEQ ID NO: 5 and SEQ ID NO: 6, respectively. Preferably, the antibody in the pharmaceutical composition of the present invention has a heavy chain variable region as shown in SEQ ID NO: 19 and a light chain variable region as shown in SEQ ID NO: 20, or has a heavy chain variable region as shown in SEQ ID NO: 21 and a light chain variable region as shown in SEQ ID NO: 20, or has a heavy chain variable region as shown in SEQ ID NO: 21 and a light chain variable region as shown in SEQ ID NO: 22; more preferably, the antibody in the pharmaceutical composition of the present invention has a heavy chain amino acid sequence as shown in SEQ ID NO: 28 and a light chain amino acid sequence as shown in SEQ ID NO: 29, respectively, or the antibody in the pharmaceutical composition of the present invention has a heavy chain amino acid sequence as shown in SEQ ID NO: 30 and a light chain amino acid sequence as shown in SEQ ID NO: 29, respectively, or the antibody in the pharmaceutical composition of the present invention has a heavy chain amino acid sequence as shown in SEQ ID NO: 30 and a light chain amino acid sequence as shown in SEQ ID NO: 31, respectively; and more preferably, the antibody in the pharmaceutical composition of the present invention has a heavy chain amino acid sequence as shown in SEQ ID NO: 28 and a light chain amino acid sequence as shown in SEQ ID NO: 29, respectively.

The humanized antibody in the pharmaceutical composition of the present invention may preferably have HCDR1, HCDR2 and HCDR3 as shown in SEQ ID NO: 7, SEQ ID NO: 8 and SEQ ID NO: 9, respectively, and LCDR1, LCDR2 and LCDR3 as shown in SEQ ID NO: 10, SEQ ID NO: 11 and SEQ ID NO: 12, respectively. Preferably, the antibody in the pharmaceutical composition of the present invention has a heavy chain variable region as shown in SEQ ID NO: 23 and a light chain variable region as shown in SEQ ID NO: 24, or has a heavy chain variable region as shown in SEQ ID NO: 23 and a light chain variable region as shown in SEQ ID NO: 25; more preferably, the antibody in the pharmaceutical composition of the present invention has a heavy chain amino acid sequence as shown in SEQ ID NO: 32 and a light chain amino acid sequence as shown in SEQ ID NO: 33, respectively, or has a heavy chain amino acid sequence as shown in SEQ ID NO: 32 and a light chain amino acid sequence as shown in SEQ ID NO: 34, respectively.

The humanized antibody in the pharmaceutical composition of the present invention may preferably have HCDR1, HCDR2 and HCDR3 as shown in SEQ ID NO: 13, SEQ ID NO: 14 and SEQ ID NO: 15, respectively, and LCDR1, LCDR2 and LCDR3 as shown in SEQ ID NO: 16, SEQ ID NO: 17 and SEQ ID NO: 18, respectively. Preferably, the antibody in the pharmaceutical composition of the present invention has a heavy chain variable region as shown in SEQ ID NO: 26 and a light chain variable region as shown in SEQ ID NO: 27; and more preferably, the antibody in the pharmaceutical composition of the present invention has a heavy chain amino acid sequence as shown in SEQ ID NO: 35 and a light chain amino acid sequence as shown in SEQ ID NO: 36, respectively.

The anti-IL-17A antibody or the antigen-binding fragment thereof in the pharmaceutical composition of the present invention has a concentration of about 1-300 mg/mL, preferably about 10-250 mg/mL, preferably about 20-200 mg/mL, more preferably 60-180 mg/mL, and more preferably about 80-150 mg/mL. As a non-limiting example; the anti-IL-17A antibody or the antigen-binding fragment thereof has a concentration of about 20 mg/mL, 30 mg/mL, 40 mg/mL, 50 mg/mL, 60 mg/mL, 70 mg/mL, 80 mg /mL, 90 mg/mL, 100 mg/mL, 110 mg/mL, 120 mg/mL, 130 mg/mL, 140 mg/mL, 150 mg/mL, 160 mg/mL, 170 mg/mL, 180 mg /mL or 200 mg/mL, preferably about 80 mg/mL or 150 mg/mL.

The buffer in the pharmaceutical composition of the present invention can be selected from acetate buffer, citrate buffer, succinate buffer and histidine buffer to provide a pH of 5.0 to 6.5, preferably 5.0 to 6.0, more preferably 5.5 ± 0.3, and more preferably about 5.5 for the pharmaceutical composition of the present invention. On the other hand, the buffer used in the pharmaceutical composition of the present invention may have a pH of 5.0-6.5, preferably 5.0-6.0, more preferably 5.5 ± 0.3, and more preferably about 5.5.

Particularly preferred buffer in the pharmaceutical composition of the present invention is histidine buffer, including histidine-hydrochloride buffer or histidine-acetate buffer, preferably histidine-hydrochloride buffer. More preferably, the histidine-hydrochloride buffer is made from histidine and histidine hydrochloride, preferably L-histidine and L-histidine monohydrochloride. In some embodiments, the histidine buffer is made from 1-20 mM of L-histidine and 1-20 mM of L-histidine monohydrochloride. In some embodiments, the histidine buffer is made from histidine and histidine hydrochloride in a molar ratio of 1 : 1 to 1 : 4. In some embodiments, the histidine buffer is made from histidine and histidine hydrochloride in a molar ratio of 1 : 1. In some embodiments, the histidine buffer is made from histidine and histidine hydrochloride in a molar ratio of 1 : 3. In some embodiments, the histidine formulation is: a histidine buffer with pH 5.5 ± 0.3, preferably about 5.5, made from 4.5 mM of L-histidine and 15.5 mM of L-histidine monohydrochloride. In some embodiments, the histidine formulation is: a histidine buffer with pH 5.5 ± 0.3, preferably about 5.5, made from 7.5 mM of L-histidine and 22.5 mM of L-histidine monohydrochloride. In some embodiments, the histidine formulation is: A histidine buffer with pH 6.0 ± 0.3, preferably about 6.0, made from 15 mM of histidine and 15 mM of histidine hydrochloride.

The histidine buffer in the pharmaceutical composition of the present invention may be a histidine-acetate buffer, preferably, the molar ratio of histidine to acetate is 1 : 1 to 1.5 : 1, preferably, such a buffer has a pH of 5.5 ± 0.3, preferably about 5.5, preferably such a buffer contains 15-20 mM of histidine and 12-15 mM of acetic acid.

Therefore, the pharmaceutical composition of the present invention may contain: Histidine-histidine hydrochloride buffer which has a pH 5.0-6.0 and a concentration of 10-30 mM in the pharmaceutical composition; and 60-180 mg/mL of the anti-IL-17A antibody or the antigen-binding fragment thereof according to any of the preceding embodiments, especially hu31, hu43, hu44, hu59, hu60 and hu250 or antigen-binding fragments thereof as described herein.

In some embodiments, the pharmaceutical composition of the present invention also contains a stabilizer. Preferably, the stabilizer is selected from one or more of arginine hydrochloride, proline, glycine, sodium chloride, mannitol, sorbitol, sucrose, maltose, xylitol and trehalose. Preferably, the stabilizer in the pharmaceutical composition contains at least arginine hydrochloride, optionally contains one or more of mannitol, sorbitol, sucrose and trehalose. For example, the pharmaceutical composition may contain arginine hydrochloride and mannitol, arginine hydrochloride and sorbitol, arginine hydrochloride and sucrose, or arginine hydrochloride and trehalose. The stabilizer in the pharmaceutical composition of the present invention has a concentration of about 10 mM to 400 mM, preferably 20 mM to 300 mM, and more preferably 30 mM to 200 mM. In some embodiments, the stabilizer is sodium chloride at a concentration of about 30-200 mM; or the stabilizer is mannitol at a concentration of about 100-300 mM, preferably 200-300 mM; or the stabilizer is sorbitol at a concentration of about 100-300 mM, preferably 200-300 mM; or the stabilizer is sucrose at a concentration of about 100-300 mM, preferably 50-250 mM; or the stabilizer is trehalose at a concentration of about 100-300 mM, preferably 200-300 mM; or the stabilizer is arginine hydrochloride at a concentration of about 30-200 mM, preferably about 60-150 mM; or the stabilizer is proline at a concentration of about 100-300 mM; or the stabilizer is glycine at a concentration of about 100-300 mM. In some embodiments, the stabilizer is a combination of about 30-200 mM of sodium chloride and about 30-200 mM of mannitol; or the stabilizer is a combination of about 30-200 mM of sodium chloride and about 30-200 mM of sucrose; or the stabilizer is a combination of about 30-200 mM of arginine hydrochloride and about 30-200 mM of mannitol; or the stabilizer is a combination of about 30-200 mM, preferably about 60-120 mM of arginine hydrochloride and about 30-200 mM, preferably about 40-80 mM of sucrose.

Thus, in some embodiments, the pharmaceutical composition of the present invention contains: histidine-histidine hydrochloride buffer which has a pH 5.0-6.0 and a concentration of 10-30 mM in the pharmaceutical composition; 60-180 mg/mL of the anti-IL-17A antibody or the antigen-binding fragment thereof according to any of the preceding embodiments, especially hu31, hu43, hu44, hu59, hu60 and hu250 or antigen-binding fragments thereof as described herein; and 20 mM-300 mM of stabilizer, preferably, the stabilizer includes at least arginine hydrochloride, optionally includes one of mannitol, sorbitol, sucrose and trehalose, preferably 30-200 mM of arginine hydrochloride, or 30-200 mM of arginine hydrochloride and 30-200 mM of mannitol, or 30-200 mM of arginine hydrochloride and 30-200 mM of sucrose. In some embodiments, the stabilizer is 30-200 mM of arginine hydrochloride and 30-200 mM of sucrose. In some embodiments, the stabilizer is 60-120 mM of arginine hydrochloride and 40-80 mM of sucrose.

In some embodiments, the pharmaceutical composition of the present invention further includes a surfactant. Preferred surfactant is selected from polysorbate 80, polysorbate 20 and poloxamer 188. The most preferred surfactant is polysorbate 20. The surfactant in the pharmaceutical composition of the present invention has a concentration of about 0.001%-0.1%, preferably about 0.02%-0.08%, and preferably about 0.02%-0.04%, on a w/v basis. As a non-limiting example, the surfactant in the pharmaceutical composition of the present invention has a concentration of about 0.02%, 0.04% or 0.08%, preferably 0.02%.

Thus, in some embodiments, the pharmaceutical composition of the present invention contains: histidine-histidine hydrochloride buffer which has a pH 5.0-6.0 and a concentration of 10-30 mM in the pharmaceutical composition; 60-180 mg/mL of the anti-IL-17A antibody or the antigen-binding fragment thereof according to any of the preceding embodiments, especially hu31, hu43, hu44, hu59, hu60 and hu250 or antigen-binding fragments thereof as described herein; 20 mM-300 mM of stabilizer, preferably, the stabilizer includes at least arginine hydrochloride, optionally includes one of mannitol, sorbitol, sucrose and trehalose, preferably 100-180 mM of arginine hydrochloride, or 60-120 mM of arginine hydrochloride and 40-80 mM of mannitol, or 60-120 mM of arginine hydrochloride and 40-80 mM of sucrose; and 0.02%-0.04% polysorbate 20 on a w/v basis.

The pharmaceutical composition of the present invention may be a liquid formulation or a lyophilized formulation.

### Medicinal uses and methods

The present invention also provides the pharmaceutical composition according to any of the embodiments of the present invention for the treatment or prevention of IL-17A-mediated diseases, use of the pharmaceutical composition according to any of the embodiments of the present invention in the preparation of a medicament for the treatment or prevention of IL-17A mediated diseases,and a method for administering a therapeutically effective amount of the pharmaceutical composition according to any of the embodiments of the present invention to an individual or patient in need thereof to treat or prevent IL-17A-mediated diseases.

In the present invention, IL-17A mediated diseases refer to diseases in which IL-17A is involved in the occurrence and development of the diseases, including but not limited to inflammatory diseases and autoimmune diseases. In the present invention, diseases suitable for the treatment and prevention using the pharmaceutical composition of the present invention include but are not limited to arthritis, rheumatoid arthritis, ankylosing spondylitis, chronic obstructive pulmonary disease, systemic lupus erythematosus (SLE), lupus nephritis, asthma, multiple sclerosis, cystic fibrosis and psoriasis.

The present invention will hereinafter be illustrated by way of specific examples. It should be understood that these examples are illustrative only and are not intended to limit the scope of the present invention. The methods and materials used in the examples, unless otherwise stated, are conventional methods and materials in the art.

### Example 1: Buffer systems and pH screening experiments

In liquid pharmaceutical compositions, the buffer system and pH closely affect the stability of antibodies, and each antibody with unique physicochemical properties has the most suitable buffer type and pH. This example aims to screen an optimal buffer system and pH, so that the anti-IL-17A antibody disclosed in the present invention has the best stability and is suitable for clinical application.

This example was performed with antibody hu31 at concentrations of about 80 mg/mL and 150 mg/mL. The samples were subjected to ultrafiltration, concentration and exchange of medium using Millipore Pellicon3 0.11m² membrane. After exchange of medium, the samples were in the corresponding formulations, and the samples were placed in a sealed centrifuge tube for buffer screening. Acetate buffer, succinate buffer and histidine buffer were screened from pH 5.0 to 6.5 (as shown in Table 3). The samples were placed in an environment of 40 ± 2°C, and were taken out for analysis and assay at the 0th, 2nd, and 4th weeks, respectively. The main pathway for protein degradation is the formation of aggregates, products of cleavage and charged variants. Size exclusion chromatography (SEC-HPLC) was used to determine the percentages of native (protein monomer) form and aggregated form, respectively, and cation exchange chromatography (CEX-HPLC) was used to determine the percentages of the antibodies in acidic form and basic form, respectively. The SEC-HPLC monomer content and CEX-HPLC main peak content after four weeks (4W) were used to fit a straight line and calculate the slope of decline (%/week) to examine the effects of different buffer systems and pH on the antibody stability of the antibody hu31. See Table 4 and Table 5 for the summarized results.

**Table 3: Formulation information for buffer systems and pH screening experiments**

| Formulation no. | pH | Buffer systems | Excipient 1 | Excipient 2 | Polysorbate 80 |
|---|---|---|---|---|---|
| 1 | 5.0 | 20 mM of acetate buffer (glacial acetic acid-sodium acetate) | 50 mM of sodium chloride | 140 mM of Mannitol | 0.02% |
| 2 | 5.5 | 20 mM of acetate buffer (glacial acetic acid-sodium acetate) | 50 mM of sodium chloride | 140 mM of Mannitol | 0.02% |
| 3 | 5.5 | 20 mM of histidine buffer (histidine-histidine hydrochloride) | 50 mM of sodium chloride | 140 mM of Mannitol | 0.02% |
| 4 | 6.0 | 20 mM of histidine buffer (histidine-histidine hydrochloride) | 50 mM of sodium chloride | 140 mM of Mannitol | 0.02% |
| 5 | 5.5 | 20 mM of histidine buffer (histidine-glacial acetic acid) | 50 mM of sodium chloride | 140 mM of Mannitol | 0.02% |
| 6 | 5.5 | 20 mM of succinate buffer (succinic acid-sodium succinate) | 50 mM of sodium chloride | 140 mM of Mannitol | 0.02% |

**Table 4: Degradation rate of SEC-HPLC monomer content for Buffer Systems and pH Screening Experiments**

| Formulation No. | 80 mg/mL protein decline rate (%/week) | 150 mg/mL protein decline rate (%/week) | Average rate (%/week) |
|---|---|---|---|
| 1 | 0.40 | 0.52 | 0.46 |
| 2 | 0.32 | 0.42 | 0.37 |
| 3 | 0.28 | 0.32 | 0.30 |
| 4 | 0.20 | 0.32 | 0.26 |
| 5 | 0.18 | 0.30 | 0.24 |
| 6 | 0.30 | 0.50 | 0.40 |

**Table 5: Degradation rate of CEX-HPLC heterogenous charged matter content for buffer systems and pH screening experiments**

| Formulation No. | 150 mg/mL protein decline rate (%/week) |
|---|---|
| 1 | 4.32 |
| 2 | 5.42 |
| 3 | 5.88 |
| 4 | 7.62 |
| 5 | 6.15 |
| 6 | 5.28 |

As shown in Table 4 and Table 5, in the SEC-HPLC experimental assay, after placed at high temperature of 40 ± 2°C for 4 weeks, the decline rate of monomer content increased with the increase of protein concentration, but in the pH 5.5 histidine buffer system, the decline rate of monomer content was low under different protein concentrations, with the average decline rate of 0.24%/week. In the CEX-HPLC assay, it can be seen that the degradation rate of the main peak content of CEX-HPLC had a certain correlation with pH. The higher the pH, the faster the degradation rate of the main peak content. In the buffer system with pH 5.0 to 5.5, the degradation rate of the main peak content is relatively low.

According to the above screening results, comprehensively considering the clinical dosage of the product, product specifications and the target quality attributes of the product (aggregate content and charge variation content level), a protein concentration of 150 mg/mL and a histidine buffer system with pH 5.5 were selected for follow-up formulation screening.

### Example 2: Stabilizer (Excipient) Screening Experiment

### 2.1 Examination of Effect of Excipients on Viscosity

To further explore the effect of different excipients on antibody stability and viscosity, we selected the formulation of one of sodium chloride, sucrose, arginine hydrochloride, proline, glycine, trehalose, sorbitol or mannitol for comparative testing. The effect of the above different excipients on viscosity was examined using a combination of 20 mM histidine buffer system and other buffer systems under the condition of same osmotic pressure, pH 5.5, and MAb hu31 concentrations of 80 mg/mL and 150 mg/mL. The specific formulation information is shown in the table. 6. Each formulation was placed under 40 ± 2°C after being subpackaged, taken out in the 4th week, analyzed and assayed for the viscosity of the formulation according to standard method, and the results are shown in Table 7.

**Table 6: Formulation information when viscosity is examined with different excipients**

| Formulation No. | Buffers | pH | Excipients | Polysorbate 80 |
|---|---|---|---|---|
| 7 | 20 mM of histidine buffer (histidine-histidine hydrochloride) | 5.5 | 240 mM of Mannitol | 0.02% |
| 8 | | | 240 mM of sorbitol | 0.02% |
| 9 | | | 220 mM of sucrose | 0.02% |
| 10 | | | 220 mM of trehalose dihydrate | 0.02% |
| 11 | | | 135 mM of sodium chloride | 0.02% |
| 12 | | | 135 mM of arginine hydrochloride | 0.02% |
| 13 | | | 240 mM of proline | 0.02% |
| 14 | | | 260 mM of glycine | 0.02% |
| 15 | 150 mM of acetate buffer (glacial acetic acid-sodium acetate) | | - | 0.02% |
| 16 | 105 mM of citrate buffer (citric acid-sodium citrate) | | - | 0.02% |
| 17 | 120 mM of succinate buffer (succinic acid-sodium succinate) | | - | 0.02% |
| 18 | 170 mM of histidine buffer (histidine-histidine hydrochloride) | | - | 0.02% |

| | | | | |
|---|---|---|---|---|
| Notes: "-" denotes no addition. | | | | |

**Table 7: Viscosity measurement data when viscosity is determined with different excipients**

| Formulation No. | Viscosity (cP) 150 mg/mL of protein | Viscosity (cP) 80 mg/mL of protein |
|---|---|---|
| 7 | 8.79 | 2.15 |
| 8 | 7.18 | 2.14 |
| 9 | 7.99 | 2.39 |
| 10 | 8.30 | 2.43 |
| 11 | 6.44 | 1.89 |
| 12 | 5.87 | 2.01 |
| 13 | 6.59 | 2.12 |
| 14 | 6.78 | 2.12 |
| 15 | 5.67 | 1.89 |
| 16 | 6.05 | 2.07 |
| 17 | 5.32 | 2.00 |
| 18 | 5.12 | 1.87 |

According to Table 7, it can be seen that the viscosity is proportional to the protein concentration. As the protein concentration increases, the viscosity increases. From the viscosity comparison using different excipients (formulation 7 to formulation 14), the viscosity obtained by using arginine hydrochloride is significantly lower than that obtained by using other excipients. The viscosity data obtained by using arginine hydrochloride corresponds to only about 65% of that obtained by using mannitol.

The viscosity obtained by using the histidine buffer system is lower than that obtained by using other buffer systems by comparing different buffer systems (formulation 15 to formulation 18). On this basis, we carried out the follow-up formulaiton design and screening.

### 2.2 Examination of Effect of Excipients on Stability

To further explore the effect of different excipients on antibody stability, we selected the formulation of one or a combination of sodium chloride, sucrose, arginine hydrochloride, trehalose, sorbitol or mannitol for comparative testing. That is, the above different excipients or their combinations were respectively added to 30 mM of histidine buffer containing about 150 mg/mL of antibody hu31. The specific formulaiton information is shown in Table 8. Each formulation was placed under 40 ± 2°C after being subpackaged, and were taken out for analysis and assay at the 0th, 2nd, and 4th weeks, respectively. The change of antibody hu31 monomer content was determined by size exclusion high performance liquid chromatography (SEC-HPLC), and the charge main peak content of antibody hu31 was determined by weak cation high performance liquid chromatography (CEX-HPLC). The results are shown in Table 9.

According to the stability examination results, the formulation samples of different excipients were placed for 4 weeks under the high temperature condition of 40 ± 2°C, and the antibodies all had strong thermal stability.

Based on the analysis of various data, the comparison of formulations 19-24 revealed that under comparison of single excipients, the single excipient containing arginine hydrochloride was obviously superior to the single excipient of sodium chloride, mannitol, sorbitol, sucrose and trehalose, as evidenced by the lower degradation rates of the monomer content of SEC-HPLC and the main peak content of CEX-HPLC. The comparison of formulations 25-30 revealed that it is most stable when using a combination of excipients arginine hydrochloride and sucrose, and the monomer content of SEC-HPLC decreased at a rate as low as 0.2%/week, which was obviously superior to that when using single excipient. There was no significant difference in the degradation rate of the main peak content of CEX-HPLC.

Therefore, the excipient combination of arginine hydrochloride and sucrose was more beneficial to the stability of the product.

**Table 8: Formulation information for excipient screening experiments**

| Formulation No. | Buffers | PH | Excipient 1 | Excipient 2 | Polysorbate 80 |
|---|---|---|---|---|---|
| 19 | 30 mM of histidine buffer (histidine-histidine hydrochloride) | 5.5 | 240 mM of Mannitol | - | 0.02% |
| 20 | | | 240 mM of D-sorbitol | - | 0.02% |
| 21 | | | 220 mM of sucrose | - | 0.02% |
| 22 | | | 220 mM of trehalose dihydrate | - | 0.02% |
| 23 | | | 135 mM of arginine hydrochloride | - | 0.02% |
| 24 | | | 135 mM of sodium chloride | - | 0.02% |
| 25 | | | 50 mM of sodium chloride | 120 mM of mannitol | 0.02% |
| 26 | | | 90 mM of sodium chloride | 50 mM of mannitol | 0.02% |
| 27 | | | 50 mM of sodium chloride | 120 mM of sucrose | 0.02% |
| 28 | | | 90 mM of sodium chloride | 50 mM of sucrose | 0.02% |
| 29 | | | 90 mM of arginine hydrochloride | 50 mM of mannitol | 0.02% |
| 30 | | | 90 mM of arginine hydrochloride | 50 mM of sucrose | 0.02% |

| | | | | | |
|---|---|---|---|---|---|
| Notes: "-" denotes no addition. | | | | | |

**Table 9: Summary of Excipient Screening Experiment Results**

| Formulation No. | SEC-HPLC Monomer decline rate (%/week) | CEX-HPLC Main peak decline rate (%/week) |
|---|---|---|
| 19 | 0.49 | 6.48 |
| 20 | 0.34 | 6.36 |
| 21 | 0.34 | 6.38 |
| 22 | 0.34 | 6.76 |
| 23 | 0.30 | 5.90 |
| 24 | 0.38 | 6.21 |
| 25 | 0.33 | 5.88 |
| 26 | 0.38 | 6.17 |
| 27 | 0.30 | 6.15 |
| 28 | 0.44 | 6.08 |
| 29 | 0.28 | 6.32 |
| 30 | 0.20 | 6.00 |

Based on the analysis of various data, the formulation containing the excipient combination of arginine hydrochloride and sucrose had the most stable antibodies and the lowest viscosity. Specifically, after being placed at a high temperature of 40 ± 2°C for 4 weeks, for the formulation group containing the combination of arginine hydrochloride and sucrose: (1) regarding the stability of antibody structure, the decline rate of antibody monomer purity was obviously the lowest, as low as 0.2%/week, which was about 40% of that of the mannitol group with the highest decline rate, and the antibody monomer purity was as high as 99.2%; (2) regarding the stability of antibody charge, the decline rate of the main charge of the antibody was low, as low as 6.00%/week, and the main charge was as high as 86.0%; (3) regarding the viscosity of the formulation, arginine hydrochloride can significantly reduce the viscosity of the formulation, the viscosity of the formulation containing only arginine hydrochloride was lower than 6 cP (Table 7), which was significantly lower than that of the other formulation groups, especially that of the formulation group containing only mannose.

Solutions with high concentration of antibodies were generally more likely to cause antibody aggregation, precipitation, *etc.,* which reduced antibody stability, and the increase in solution viscosity made injection (especially subcutaneous or intramuscular injection) difficult to administer. Based on the above experiments, we found that in the liquid formulation, arginine salt can not only ensure antibody stability, but also significantly reduce liquid viscosity. In particular, when the buffer was histidine buffer with pH 5.5 and the stabilizer contained arginine hydrochloride and sucrose, the effect for antibody stability and solution viscosity was optimal.

### Example 3: Surfactant Screening Experiment

The addition of surfactants to liquid formulations is often used to protect proteins such as antibodies from air/solution interface induced stress, solution/surface induced stress to reduce aggregation of antibodies or to minimize the formation of particulate matter in the formulation, which is beneficial to the stability of the physicochemical properties of the antibodies. Different concentrations of polysorbate 20 or polysorbate 80 were respectively added to formulations containing 30 mM of histidine buffer (histidine-histidine hydrochloride, pH 5.5) and 150 mg/mL of antibody hu31 (the excipient 1 and the excipient 2 were added according to the formulation 30), and placed at 40 ± 2°C for 2 weeks. 4 weeks later, analysis and assay were performed, a straight line was fitted, and the slope of decline (%/week) was calculated, and the results are shown in Table 10.

The comprehensive analysis revealed that the results of the surfactant screening experiment (formulations 31-36) showed that the addition of different concentrations of polysorbate 80 or polysorbate 20 had no obvious effect on the monomer content of SEC-HPLC.

**Table 10: surfactant screening results**

| Formulation No. | Surfactant content | SEC-HPLC (%) Monomer decline rate (%/week) |
|---|---|---|
| 31 | 0.02% polysorbate 20 | 0.37 |
| 32 | 0.04% polysorbate 20 | 0.32 |
| 33 | 0.08% polysorbate 20 | 0.32 |
| 34 | 0.02% polysorbate 80 | 0.30 |
| 35 | 0.04% polysorbate 80 | 0.35 |
| 36 | 0.08% polysorbate 80 | 0.42 |

### Example 4: Formulation Dilution Stability and Formulation Confirmation

An antibody stock solution was selected and the formulation No. 30 as shown in Table 8 was formulated. Samples were diluted with normal saline, glucose injection and different formulations to different concentrations to examine the stability of the samples. Meanwhile, the stability of the samples under the repeated freeze-thaw conditions was examined to confirm the formulation conditions (Table 11). The screening results are shown in Table 12.

**Table 11: Examination information (formulation 30)**

| | |
|---|---|
| Repeated freeze-thaw | Freeze-thaw for 5 times at -40 ± 5°C |
| Dilution with normal saline | Diluting the concentration to 10 mg/mL |
| Glucose injection | Diluting the concentration to 10 mg/mL |
| Formulation 30 Buffer | Diluting the concentration to 2 mg/mL, 10 mg/mL |

**Table 12: Formulation stability screening results**

| Examination conditions | Concent ration (mg/ml) | Dilution medium | Appearance examination | Monomer content (%) | Insoluble particles | |
|---|---|---|---|---|---|---|
| | | | | | ≥ 10 um (particles/ ml) | ≥ 25 um (particles /ml) |
| Repeated freeze-thaw for 0 times | 150 | / | Conformity with standards | 99.7 | / | / |
| After repeated freeze-thaw for 5 times | 150 | / | Conformity with standards | 99.7 | / | / |
| Fomulation 30 | 150 | / | Conformity with standards | / | 0 | 0 |
| Fomulation 30 | 10 | 0.9% sodium chloride | Conformity with standards | / | 0 | 0 |
| Fomulation 30 | 10 | 5% glucose | Conformity with standards | / | 10 | 0 |
| Fomulation 30 | 10 | Formula tion 30 Buffer | Conformity with standards | / | 8 | 0 |
| Fomulation 30 | 2 | Formula tion 30 Buffer | Conformity with standards | / | 1 | 0 |

| | | | | | | |
|---|---|---|---|---|---|---|
| Notes: "/" denotes no measurement or no addition. | | | | | | |

Under this formulation condition, repeated freeze-thaw at 25 ± 2°C of exampes and dilution with glucose injection, normal saline and formulation 30 buffer (*i.e.* 30 mM of histidine buffer) of exampes to different concentrations have no significant influence on the monomer content, which showed the good stability of the formulation.

In summary, we explored and studied the stability of recombinant humanized anti-IL-17A monoclonal antibody hu31 and determined the best liquid formulation by investigating different buffer systems, different pH conditions, different antibody concentrations, and different compositions of excipients and surfactants. For antibody hu31, histidine buffer was selected to adjust pH, arginine hydrochloride and sucrose were selected to adjust formulation osmotic pressure and viscosity, and polysorbate 20 was added to increase formulation solubility.

### Example 5: Long-term stability study of formulation

Liquid pharmaceutical products containing therapeutic antibodies usually need to be stored at 2-8°C, so it is very important that the formulations maintain high stability during long-term storage. According to the above screening results, we selected 4 batches of antibody stock solutions and formulation No. 31 as shown in Table 13 was formulated for long-term stability study of the formulation.

It was placed at 2~8°C for 12 months, and analyzed and assayed for each sample. Stability was assessed by the following parameters: (a) the molecular weight of the antibody determined by CE-SDS (sodium dodecyl sulfate capillary electrophoresis) method; (b) the content of antibody monomers (quality standard: ≥ 97.0%), the content of aggregates (quality standard: ≤ 3.0%) or fragments (quality standard: ≤ 1.0%) measured by SEC-HPLC; (c) the content of the main charge (quality standard: ≥ 70.0%), acidic charge (quality standard: ≤ 30.0%) or basic charge (quality standard: ≤ 15.0%) of the antibody measured by CEX-HPLC; biological activity (quality standard: 70%-130% of the reference product).

The results show that when using formulation No. 31, the purity (SEC-HPLC (molecular exclusion high performance liquid chromatography), CEX-HPLC (weak cation exchange high performance liquid chromatography), R-CE-SDS (reduction electrophoresis), NR-CE-SDS (non-reduction electrophoresis)) and biological activity of the 4 batches of stock solutions have no significant change. See Table 14 for details. The results show that the above 4 batches of stock solutions have very good stability during storage for 0-12 months at 2-8°C when using formulation No. 31.

**Table 13: Formulation for long-term stability studies**

| Formulation No. | pH | Formulation composition |
|---|---|---|
| 31 | 5.5 | 30 mM of histidine buffer, 90 mM of arginine hydrochloride, 50 mM of sucrose, 0.02% polysorbate 20; 150 mg/mL of antibody hu31 |

**Table 14: Long-term stability data for formulation**

| Test item | time (month) | Test results | | | |
|---|---|---|---|---|---|
| | | Batch 1 | Batch 2 | Batch 3 | Batch 4 |
| SEC-HPLC monomer content (%) | 0 | 99.6% | 99.7% | 99.8% | 99.9% |
| | 1 | 99.8% | 99.6% | 99.7% | NA |
| | 2 | 99.6% | 99.8% | 99.7% | NA |
| | 3 | 99.6% | 99.7% | 99.7% | 99.8% |
| | 6 | 99.6% | 99.7% | 99.7% | 99.8% |
| | 9 | 99.5% | 99.7% | 99.6% | 99.7% |
| | 12 | 99.4% | 99.5% | 99.5% | 99.8% |
| SEC-HPLC aggregate content (%) | 0 | 0.4% | 0.2% | 0.2% | 0.1% |
| | 1 | 0.2% | 0.4% | 0.3% | NA |
| | 2 | 0.4% | 0.2% | 0.3% | NA |
| | 3 | 0.4% | 0.2% | 0.3% | 0.2% |
| | 6 | 0.4% | 0.3% | 0.3% | 0.2% |
| | 9 | 0.5% | 0.3% | 0.4% | 0.3% |
| | 12 | 0.6% | 0.4% | 0.4% | 0.2% |
| SEC-HPLC fragment ccontent (%) | 0 | 0.0% | 0.0% | 0.0% | ND |
| | 1 | 0.0% | 0.0% | 0.0% | NA |
| | 2 | 0.0% | 0.0% | 0.0% | NA |
| | 3 | 0.0% | 0.0% | 0.0% | ND |
| | 6 | 0.0% | 0.0% | 0.0% | < 0.1% |
| | 9 | 0.0% | 0.0% | 0.0% | ND |
| | 12 | 0.0% | 0.0% | 0.0% | ND |
| CEX-HPLC main peak content (%) | 0 | 78.2% | 77.1% | 77.1% | 77.2% |
| | 1 | 78.0% | 78.1% | 77.6% | NA |
| | 2 | 78.3% | 78.4% | 78.0% | NA |
| | 3 | 77.9% | 77.6% | 77.0% | 77.4% |
| | 6 | 78.6% | 78.8% | 78.3% | 77.9% |
| | 9 | 77.3% | 77.4% | 76.9% | 76.5% |
| | 12 | 76.6% | 77.0% | 76.6% | 74.9% |
| CEX-HPLC acidic peak content (%) | 0 | 16.6% | 15.9% | 17.2% | 16.2% |
| | 1 | 16.1% | 16.6% | 16.9% | NA |
| | 2 | 16.2% | 15.6% | 16.5% | NA |
| | 3 | 16.4% | 16.1% | 17.1% | 16.2% |
| | 6 | 17.1% | 16.6% | 17.4% | 16.2% |
| | 9 | 17.4% | 16.9% | 17.9% | 17.4% |
| | 12 | 18.5% | 17.8% | 18.8% | 19.3% |
| CEX-HPLC basic peak content (%) | 0 | 5.1% | 6.3% | 5.6% | 6.6% |
| | 1 | 5.9% | 5.4% | 5.4% | NA |
| | 2 | 5.5% | 6.0% | 5.4% | NA |
| | 3 | 5.7% | 6.3% | 5.9% | 6.4% |
| | 6 | 4.3% | 4.6% | 4.2% | 5.9% |
| | 9 | 5.2% | 5.7% | 5.3% | 6.1% |
| | 12 | 4.9% | 5.2% | 4.6% | 5.8% |
| R-CE-SDS content (%) | 0 | 100.0% | 99.9% | 99.9% | 99.7% |
| | 1 | 99.8% | 99.8% | 99.8% | NA |
| | 2 | 99.8% | 100.0% | 99.9% | NA |
| | 3 | 99.7% | 99.6% | 99.6% | 99.8% |
| | 6 | 99.7% | 99.7% | 99.7% | 99.7% |
| | 9 | 99.6% | 99.7% | 99.7% | 99.8% |
| | 12 | 99.7% | 99.7% | 99.7% | 99.8% |
| NR-CE-SDS content (%) | 0 | 99.5% | 99.4% | 99.4% | 97.7% |
| | 1 | 99.2% | 99.4% | 99.4% | NA |
| | 2 | 99.4% | 99.3% | 99.4% | NA |
| | 3 | 99.4% | 99.4% | 99.4% | 98.0% |
| | 6 | 99.0% | 98.9% | 98.9% | 98.9% |
| | 9 | 99.8% | 99.7% | 99.7% | 99.1% |
| Biological activity (Blocking ELISA method), % | 12 | 99.6% | 99.6% | 99.7% | 98.8% |
| | 0 | 104.8% | 93.2% | 102.1% | 100% |
| | 1 | 94.3% | 99.3% | 100.5% | NA |
| | 2 | 106.9% | 101.0% | 102.5% | NA |
| | 3 | 100.6% | 99.3% | 97.8% | 102% |
| | 6 | 97.5% | 101.9% | 105.2% | 101% |
| | 9 | 97.9% | 120.0% | 104.8% | 104% |
| | 12 | 96.7% | 96.0% | 101.3% | 99% |
| Biological activity (Binding ELISA method), % | 0 | 100.8% | 108.0% | 109.2% | 108% |
| | 6 | 99.8% | 103.1% | 101.3% | 110% |
| | 12 | 100.1% | 92.5% | 100.0% | 106% |
| Biological activity (bioluminescence-based cell activation assay), % | 0 | 107.4% | 101% | 100% | 98% |
| | 1 | NA | NA | NA | 99% |
| | 2 | NA | NA | NA | NR |
| | 3 | NA | 95% | 100% | 101% |
| | 6 | 106.1% | 100% | 102% | 105% |
| | 9 | NA | 96% | 99% | 103% |
| | 12 | 109.7% | 95% | 100% | NR |

| | | | | | |
|---|---|---|---|---|---|
| Notes: In the table, NA denotes that it is a non-key test item at this time point, and no test is performed at this sampling point; NR denotes that the time point has not been reached, the same below; ND denotes not detected. | | | | | |

### Example 6: Accelerated stability study of formulation

4 batches of stock solutions were selected, and formulation No. 31 as shown in Table 13 was formulated, stored in transparent vials respectively, and placed at 25 ± 2°C and 60 ± 5% relative humidity (RH). After 0-6 months, analysis and assay were performed on each sample. Stability was assessed by the following parameters: (a) the molecular weight of the antibody determined by CE-SDS (sodium dodecyl sulfate capillary electrophoresis) method; (b) the content of antibody monomers (quality standard: ≥ 97.0%), aggregates (quality standard: ≤ 3.0%) or fragments (quality standard: ≤ 1.0%) measured by SEC-HPLC; (c) the content of the main charge (quality standard: ≥ 70.0%), acidic charge (quality standard: ≤ 30.0%) or basic charge (quality standard: ≤ 15.0%) of the antibody measured by CEX-HPLC; (d) biological activity (quality standard: 70%-130% of the reference product). See Table 15 for details.

Stored under accelerated stability conditions (25 ± 2°C, 60 ± 5% RH) for 6 months, the main peak of CEX-HPLC dropped, the acid peak rised and exceeded the quality standard by 6 months; the monomer content of SEC-HPLC and the purity of R-CE-SDS tended to decrease, but did not exceed the quality standard; there was no significant change in biological activity.

**Table 15: Accelerated stability data for formulation**

| Test item | time (month) | Test results | | | |
|---|---|---|---|---|---|
| | | Batch 1 | Batch 2 | Batch 3 | Batch 4 |
| SEC-HPLC monomer (%) | 0 | 99.6% | 99.7% | 99.8% | 99.9% |
| | 1 | 99.5% | 99.7% | 99.8% | 99.8% |
| | 2 | 99.5% | 99.6% | 99.6% | 99.7% |
| | 3 | 99.3% | 99.5% | 99.4% | 99.7% |
| | 6 | 99.2% | 99.4% | 99.3% | 99.5% |
| SEC-HPLC aggregate (%) | 0 | 0.4% | 0.2% | 0.2% | 0.1% |
| | 1 | 0.5% | 0.3% | 0.2% | 0.2% |
| | 2 | 0.5% | 0.4% | 0.4% | 0.2% |
| | 3 | 0.6% | 0.4% | 0.5% | 0.3% |
| | 6 | 0.7% | 0.5% | 0.6% | 0.4% |
| SEC-HPLC fragment (%) | 0 | 0.0% | 0.0% | 0.0% | ND |
| | 1 | 0.0% | 0.0% | 0.0% | ND |
| | 2 | 0.0% | 0.0% | 0.0% | 0.1% |
| | 3 | 0.0% | 0.1% | 0.0% | ND |
| | 6 | 0.0% | 0.0% | 0.0% | <0.1% |
| CEX-HPLC main peak (%) | 0 | 78.2% | 77.1% | 77.1% | 77.2% |
| | 1 | 74.8% | 74.5% | 74.0% | 73.6% |
| | 2 | 71.8% | 71.7% | 71.4% | 67.2% |
| | 3 | 68.1% | 67.8% | 67.1% | 65.9% |
| | 6 | 61.6% | 61.5% | 61.0% | 56.3% |
| CEX-HPLC acidic peak (%) | 0 | 16.6% | 15.9% | 17.2% | 16.2% |
| | 1 | 19.9% | 19.7% | 20.6% | 21.4% |
| | 2 | 22.8% | 22.8% | 23.4% | 25.6% |
| | 3 | 26.4% | 25.9% | 26.7% | 27.7% |
| | 6 | 34.6% | 34.5% | 35.2% | 36.8% |
| CEX-HPLC basic peak (%) | 0 | 5.1% | 6.3% | 5.6% | 6.6% |
| | 1 | 5.4% | 5.8% | 5.4% | 5.0% |
| | 2 | 5.4% | 5.5% | 5.2% | 7.1% |
| | 3 | 5.6% | 6.3% | 6.2% | 6.4% |
| | 6 | 3.9% | 4.0% | 3.7% | 6.9% |
| R-CE-SDS (%) | 0 | 100.0% | 99.9% | 99.9% | 99.7% |
| | 1 | 99.8% | 99.8% | 99.9% | 99.5% |
| | 2 | 99.7% | 99.8% | 99.7% | 99.6% |
| | 3 | 99.4% | 99.5% | 99.4% | 99.3% |
| | 6 | 98.7% | 98.6% | 98.7% | 98.4% |
| NR-CE-SDS (%) | 0 | 99.5% | 99.4% | 99.4% | 97.7% |
| | 1 | 99.2% | 99.2% | 99.3% | 99.4% |
| | 2 | 99.2% | 99.1% | 99.1% | 99.1% |
| | 3 | 99.1% | 99.1% | 99.1% | 99.0% |
| | 6 | 97.5% | 97.7% | 97.6% | 98.4% |
| Biological activity (Blocking ELISA method), %) | 0 | 104.8% | 93.2% | 102.1% | 100% |
| | 1 | 98.9% | 93.7% | 96.1% | 95% |
| | 2 | 97.8% | 104.0% | 111.3% | 96% |
| | 3 | 105.1% | 88.6% | 98.1% | 99% |
| | 6 | 90.6% | 98.6% | 99.6% | 100% |
| Biological activity (Binding ELISA method), % | 0 | 100.8% | 108.0% | 109.2% | 108% |
| | 6 | 96.9% | 95.3% | 95.3% | 100% |
| Biological activity (bioluminescence-based cell activation assay), % | 0 | 97.9% | 96.6% | 81.7% | 105% |
| | 1 | 77.5% | 122.8% | 108.5% | 121% |
| | 2 | 104.7% | 87.9% | 112.3% | 99% |
| | 3 | 101.2% | 102.5% | 98.8% | 103% |
| | 6 | 83.2% | 73.8% | 81.2% | 100% |

| | | | | | |
|---|---|---|---|---|---|
| Notes: In the table, NA denotes that it is a non-key test item at this time point, and no test is performed at this sampling point; NR denotes that the time point has not been reached; ND denotes not detected. | | | | | |

### Example 7: Binding specificity of humanized antibodies to human IL-17A

The binding specificity of different humanized antibodies (formulated according to formulation No. 31) to human IL-17A was detected by conventional ELISA detection method. That is, a 96-well microtiter plate was coated by 0.5 µg/ml of human IL-17A-mFc, and incubated at a constant temperature of 37°C for 60-90 minutes. Then, the solution in the well was discarded, and the well was washed 3 times with washing buffer, and blocked for 60 minutes by adding a PBS solution containing 2% BSA. After washing 3 times with washing buffer, diluents of humanized antibodies with different concentrations were added, incubated at 37°C for 60 minutes and washed 3 times with washing buffer; and then 1 : 10,000-fold diluted biotin-anti-IgG4 was added, incubated at 37°C for 1 hour and washed three times with washing buffer; and then HPR-Strep diluted by 1 : 10,000-fold with washing buffer was added, incubated at room temperature for 1 hour and washed with washing buffer 3 times; and then 100 µL of TMB substrate solution was added to develop color; after 30 minutes of reaction at room temperature, the reaction was terminated with 100 µL of 2 M hydrochloric acid solution and the absorbance was read at 450 nm. As shown in Fig. 1, the humanized antibodies hu31, hu43, hu44, hu59, hu60 and hu250 all specifically bound to IL-17A. Their EC50s were 8.13 ng/mL, 8.64 ng/mL, 6.76 ng/mL, 6.10 ng/mL, 5.78 ng/mL and 6.35 ng/mL, respectively.

### Example 8: Blocking of the binding of human IL-17A to IL-17RA by humanized antibodies

Competitive cell-based flow cytometry (FACS) was used to detect blocking of the binding of IL-17A to IL-17RA on cells by different humanized antibodies (formulated according to formulation no. 31). Briefly, diluents of humanized antibodies with different concentrations (starting from 10 ug/ml, 3-fold titration) were mixed with pre-biotinylated human IL-17A-mFC (3 ug/ml) obtained in Example 1 and incubated at room temperature for 30 minutes. The mixture was then incubated with the cell suspension (stably transfected 293F IL-17RA cell line obtained in Example 2, 1.5 × 10⁵ cells/well) at 37°C for 15 minutes, and after eluting with PBS 3 times, 5 µg/ml of anti-mouse IgG was added and incubated at room temperature for 30 minutes. After eluting with PBS 3 times, the inhibitory effect of the humanized antibodies on the binding of IL-17A to IL-17RA on the surface of 293F cells was detected by flow cytometry. As shown in Fig. 2, the humanized antibodies hu31, hu43, hu44, hu59, hu60 and hu250 could significantly inhibit the specific binding of IL-17A to IL-17RA on cells. Their IC50s are 867.6 ng/mL, 780.8 ng/mL, 828.5 ng/mL, 467.4 ng/mL, 482.8 ng/mL and 577.8 ng/mL, respectively.

### Example 9: Humanized antibodies antagonize IL-17A-induced CXCL1 expression by epithelial cells

IL-17A can stimulate various epithelial cells and other cells to express and secrete and release cytokine CXCL1. The change in the expression of CXCL1 in the cell supernatant can be quantitatively detected by ELISA to determine the effect of different humanized antibodies (formulated according to formulation No. 31) on the biological activity mediated by IL-17A in cells.

HT-29 cells (human colorectal adenocarcinoma epithelial cells, ATCC) were maintained in culture/assay medium in tissue culture-treated flasks using standard techniques. HT-29 were grown in tissue culture flasks until they reached 50-80% confluence on the day of the assay. On the day of the assay, the cells were washed with PBS and detached from the culture flasks with trypsin + EDTA and made into a cell suspension. Humanized antibody hu31, hu43, hu44, hu59, hu60 and hu250 or reference antibody (Secukinumab, Novartis) diluents (initial concentration of 55 ug/ml, 3-fold concentration gradient dilution) were taken and mixed with human IL-17A (1 ug /ml), mix, plated into a 96-well plate, and incubated for 1 h. 100 ul (2 × 10⁴) of HT-29 cell (ATCC, human colorectal adenocarcinoma epithelial cells) suspension was added to each well and cultured at 37°C, 7% CO₂ for 48 h. After centrifugation, the culture supernatant was transferred to a new 96-well plate, and the expression of CXCL1 was detected by ELISA kit.

As shown in Fig. 3, the humanized antibodies hu31, hu43, hu44, hu59, hu60 and hu250 had stronger antagonistic effects on IL-17A-stimulated CXCL1 release from epithelial cells than the reference antibody Secukinumab.

### Example 10: Humanized antibodies antagonize IL-17A-induced CXCL1 expression in mice

The change of serum CXCL1 level in mice was detected to determine the effect of different humanized antibodies (formulated according to formulation No. 31) on the biological activity mediated by IL-17A *in vivo.* Briefly, 40 10-week-old female Balb/c mice were selected and divided into 8 groups, with 5 mice in each group. 4 days before administration, serum was collected, and the expression quantity of CXCL1 was determined as the baseline value. On the day of administration, the candidate antibodies (humanized antibodies hu31, hu43, hu44, hu60 and hu250) and IgG4 isotype control (hIgG) were injected intracardially at 1 mg/kg; 1 Hour after administration, human IL-17A was injected subcutaneously at a dosage of 150 ug/kg; 2 Hours after the injection of human IL-17A, serum was collected to determine the concentration of CXCL1 in the blood which was compared with the baseline value, and the fold change of the concentration of CXCL1 in each group before and after administration was calculated (mean ± standard error). Comparative analysis of candidate antibodies with IgG4 isotype control was performed using Student's-t test, P < 0.05 was considered to be significantly different, *P < 0.05, ^{∗∗}P < 0.01, ***P < 0.001.

As shown in Fig. 4, the candidate humanized antibodies hu31, hu43, hu44, hu60 and hu250 had stronger antagonistic effects on IL-17A-stimulated CXCL1 release *in vivo* in mice than IgG4 isotype control.

### Example 11: Efficacy study of humanized antibodies in improving imiquimod-induced psoriasis in mouse model

Applying imiquimod to the skin on the back of the ear of mice can induce psoriasis-like pathological features, namely excessive proliferation of keratinocytes, aggregation of inflammatory cells and vascular proliferation in the dermal papilla, to establish a mouse model of psoriasis. The therapeutic effect of the drug on psoriasis in mice was determined by using indicators such as clinical scores, ear swelling, *etc.*

### 1. Experimental method

48 6-8 week old C57BL/6 female mice (purchased from the Model Animal Research Center of Nanjing University, animal certificate number: 201605578) were taken, and their backs were depilated. Except for the sham group ("sham"), they were sensitized two days later. Two days before sensitization, they were randomly divided into 6 groups (8 animals in each group): Group I was the sham group; Group II was the PBS group, and group III was the KLH control group (isotype IgG) dosed with KLH; Group IV was the hu31 group; Group V was the hu43 administration group; Group VI was the hu44 administration group, and the administration dose for each group was 50 mg/kg; The above groups were given intraperitoneal injection of drugs once on the 0th and 3rd day of grouping. On the day of sensitization (day 1), the mice in groups II-VI were smeared with about 62.5 mg of imiquimod cream (Adalar, 5%, 3M Health Care Limited) on the skin of the right ear and the back for 4 consecutive days. Humanized antibodies were formulated according to formulation No. 31.

### 2. Evaluation method

From the day of sensitization, the thickness of the right ear of the mice was measured with a spiral micrometer every day, and the thickness of the right ear of the mice on the first day was used as a control to calculate the thickness of the ear swelling of the mice. At the same time, the mice were weighed every day, and scaling, induration, and erythema of the skin were observed and scored using a 4-level scoring method: 0 points, no disease; 1 point, slight; 2 points, moderate; 3 points, serious; 4 points, very serious. The results were expressed as mean ± standard error. One-way analysis of variance (ANOVA) was used first, and if there was a difference between two groups, then Student's-t test was used for the comparation of the two groups. P < 0.05 was considered significant difference.

As shown in Fig. 5-1, administration of the humanized antibodies of the present invention can significantly inhibit the scaling, induration, and red and swollen of the skin of the mouse model with imiquimod-induced psoriasis, that is, the scores were low.

As shown in Fig. 5-2, from the day of sensitization, imiquimod cream was applied to the right ear of the mice, the right ear would be severely swollen, and the thickness of the ear would increase. The humanized antibodies of the present invention could significantly improve the degree of ear swelling.

In the mouse model with imiquimod-induced psoriasis, the humanized antibodies of the present invention can significantly resist the onset of disease of the mouse, and the phenotype was the reduction of the mouse clinical score and the degree of ear swelling.

Example 12: Efficacy study of humanized antibodies in improving collagen type II-induced arthritis in female cynomolgus monkeys

Type II collagen-induced arthritis is an animal model widely used in the study of rheumatoid arthritis (RA), has the same histopathological features as human RA and is characterized by inflammation of the facet joints and progressive erosion of cartilage and bone. Human/humanized biological macromolecules, including antibodies, often have better cross-reaction with antigens in cynomolgus monkeys, so the cynomolgus monkey model with arthritis is an effective system for determining the anti-rheumatic effect of humanized antibody IL-17A of the present invention. In this experiment, the efficacy of the candidate antibodies was evaluated on the cynomolgus monkey model with rheumatoid arthritis.

### 1. Experimental method

Type II bovine collagen (CII, Sichuan University) was dissolved in acetic acid (Cat. No. 10000218; Sinopharm; Shanghai; China), placed in a refrigerator at 4°C and stirred overnight, and then the collagen was emulsified with an equal volume of complete Freund's adjuvant (Cat. No. F5881, Sigma-Aldrich, USA), the final concentration of the collagen in emulsion was 2 mg/ml. On day 0, the animals were anesthetized with Zoletil (1.5-5 mg/kg, i.m.), and collagen emulsion was injected at multiple sites of the back and tail root for immunization. As needed, 1.5%-5% isoflurane was used to maintain anesthesia during immunization. 3 weeks later (day 21), the collagen was re-injected, the injection method was the same as the first time. In this experiment the animals were divided into 4 groups, G1 was the normal animal group ("naive"), and no arthritis was induced; G2 was the vehicle control group ("vehicle"); G3 was the antibody hu31 administration group; G4 was the antibody hu59 administration group. When the clinical score of an animal reached 5% of the maximum clinical score (192 × 5% ≈ 10), it would be assigned to respective experimental group in order, and the animal that reached this score first was assigned to the group first, and this cycle would continue until all eligible animals were assigned to respective groups in order. Administration was started after being assigned to respective groups, 7.5 mg/kg once a week, the dose was continuously pumped into animals by infusion pump over 30 minutes and such administration was carried out for 5 weeks. Humanized antibodies were formulated according to formulation No. 31.

### 2. Evaluation method

Body weight measurement: Animal body weight was measured the day before immunization and weekly thereafter until the end of the experiment.

Arthritis score: The degree of inflammation of arthritis in the limbs of monkeys was scored on days 0 and 21, and was scored weekly after day 21 until the end of the experiment (if there was early onset, the weekly arthritis scoring would be advanced accordingly). Scoring criteria for arthritis: 0 points, normal; 1 point, mild arthritis, mild but clearly distinguishable; 2 points, moderate swelling; 3 points, severe arthritis, severe swelling or significant joint deformation. The following 15 joints were scored for each paw: 5 metacarpophalangeal joints (MCP), 4 proximal phalangeal joints (PIP), 5 distal phalangeal joints (DIP), 1 wrist or ankle j oint. It was also necessary to assess the disease degree of the knee/elbow joints of the limbs. The sum of the individual joint scores was the arthritis score for this animal, with a maximum score of 192 (16 × 3 × 4).

Experimental data was expressed as mean ± standard error (mean ± S.E.M). Statistical analysis was made on the differences of parameters among vehicle control group, reference drug group and test drug group, p < 0.05 was considered to be statistically significant (One-way ANOVA/Dunnett).

As shown in Fig. 6-1, normal cynomolgus monkeys (G1) have stable body weight; In cynomolgus monkeys after arthritis induction, the mean body weight of the cynomolgus monkeys in the vehicle-treated group (G2) continued to decrease. In contrast, the test antibodies hu31 and hu59 could control this downward trend. Therefore, under conditions in this experiment, hu31 and hu59 had a certain improvement effect on weight loss caused by arthritis (^{∗∗}P < 0.01, ^{∗∗∗∗}P < 0.0001, compared with "G2: vehicle group"; One-way ANOVA/Dunnett).

As shown in Fig. 6-2, after the animals were assigned into groups, the arthritis clinical scores in normal control animals (G1) remained at 0; The arthritis scores in animals of the vehicle control group (G2) increased gradually, while the test antibodies hu31 and hu59 significantly inhibited the increasing trend of the arthritis clinical scores in the animals. Therefore, the test antibodies hu31 and hu59 had the effect of inhibiting the progressive development of arthritis, and the test antibody hu31 significantly inhibited the increasing trend of the arthritis clinical scores in cynomolgus monkeys (^{∗∗∗}P < 0.001, #P < 0.05, compared with vehicle group; One-way ANOVA/Dunnett).

Example 13: Effect of humanized antibodies on NIH3T3-IL17 cell-induced joint swelling in mice
1. Animals: C57BL/6, female, 6-8 weeks, Beijing Vital River Laboratory Animal Technology Co., Ltd.
2. Cells NIH3T3 cells, NIH3T3 cells expressing human IL-17.
3. Grouping and dosing schedule:
   NIH3T3 group;
   NIH3T3-IL-17 + control IgG antibody group (30 mg/kg);
   NIH3T3-IL-17 + test antibody high-dose administration group (antibody hu31, 3 mg/kg);
   NIH3T3-IL-17 + test antibody medium-dose administration group (antibody hu31, 10 mg/kg);
   NIH3T3-IL-17 + test antibody low-dose administration group (antibody hu31, 30 mg/kg);
   NIH3T3-IL-17 + positive drug administration group (cosentyx, 10 mg/kg).
   Humanized antibodies were formulated according to formulation No. 31.
4. Modeling and dosing:
   NIH3T3-IL-17 cells and NIH3T3 control cells (2.5 × 10⁵ cells/mouse, with an injection volume of 25 uL for each mouse) were injected into the joint cavity of the right ankle joint of mice in each group, respectively.
   Interventional antibody hu31 (3, 10, 30 mg/kg) and cosentyx (10 mg/kg) were administered by intraperitoneal injection 1 day before model selection, once every 3 days, to examine the effect of antibody hu31 injection on mouse model with arthritis.
5. Detection:
   Vernier calipers were used to measure the thickness of mouse ankle joints and calculate the degree of swelling.
6. Experimental results:
   As shown in Fig. 7, when NIH3T3 cells stably transfected with hIL-17 were injected into the ankle joint cavity of mice, severe swelling of ankle joint of mice was observed on the second day.

The swelling inhibition rate was calculated according to the thickness of the mouse ankle j oint, and the calculation formula was: inhibition rate (%) = (ankle joint thickness of NIH3T3-IL17 group-ankle joint thickness of administration group)/(ankle joint thickness of NIH3-II,17 group-ankle joint thickness of NIH3T3 group) × 100. The results showed that on the 2nd day after administration, the inhibitory effect of each dose group on the swelling of mouse ankle joint was observed until the end of the test, and the maximum inhibitory rate was reached on the 6th day after administration. On the 10th day, the inhibition rates of the antibody hu31 (3, 10, 30 mg/kg) groups were 49.4%, 65.9% and 74.1%, respectively. The swelling inhibition rate of cosentyx (10 mg/kg) was 67.1%. The average inhibition rate of different number of days for each group was calculated, and the results showed that the average inhibition rates for each dose group (3, 10, and 30 mg/kg) of antibody hu31 were 45.2%, 57.0%, and 73.9%, respectively. The swelling inhibition rate of cosentyx (10 mg/kg) was 60.4%. The experimental results suggested: antibody hu31 could dose-dependently inhibit IL-17-induced swelling of mouse ankle joint, and its effect of 10 mg/kg was comparable to the positive control drug cosentyx (10 mg/kg). The effect of 30 mg/kg was better than that of the positive control drug cosentyx (10 mg/kg).

Example 14: Effect of humanized antibodies on NIH3T3-IL17 cell-induced air sac inflammation in mice
1. Animals: C57BL/6, male, 6-8 weeks, purchased from Beijing Vital River Laboratory Animal Technology Co., Ltd).
2. Cells NIH3T3 cells, NIH3T3 cells expressing human IL-17.
3. Reagents: Gr1-FITC antibody, Biolegend.
4. Grouping and dosing schedule:
   NIH3T3 cell group
   NIH3T3-IL-17 cell group + control IgG antibody group (30 mg/kg);
   NIH3T3-IL-17 cell group + test antibody high-dose administration group (antibody hu31, 30 mg/kg);
   NIH3T3-IL-17 cell group + test antibody medium-dose administration group (antibody hu31, 10 mg/kg);
   NIH3T3-IL-17 cell group + test antibody low-dose administration group (antibody hu31, 3 mg/kg);
   administration route of: intraperitoneal injection, humanized antibodies were formulated according to formulation No. 31.
5. Modeling:
   air sac: mice were injected with 2.5 ml of air on the back on day 0 and day 3, respectively; the injection of cells into the air sac was started on day 5; the number of cells injected per mouse was 2 × 10⁵ cells per 500 µl of PBS; and there were 8 mice per group.
6. Detection:
   leukocytes migrated to the air sac, the total number of cells in the lavage fluid was calculated, the proportion of Gr1+ cells was determined by flow cytometry, and the number of neutrophils was calculated; among them, the number of neutrophils = the total number of cells × the proportion of Gr1+ cells.
7. Experimental results

As shown in Fig. 8, NIH-3T3 cells stably transfected with hlL-17A were injected into the air sac on the back of mice. From the total number of infiltrated cells, compared with the NIH3T3 cell group, the number of infiltrated leukocytes in the air sac of NIH3T3-IL-17 cell group was significantly increased, and the proportion and number of Gr1+ cells also increased significantly, and the model selection was successful. On the day of modeling, interventional antibody hu31 (dose of 3, 10, and 30 mg/kg) was administered by intraperitoneal injection. The inhibition rate was calculated according to the total number of infiltrated cells and the number of Gr1+ cells, and the calculation formula was: inhibition rate (%) = (number of cells in NIH3T3-IL17-IgG group - number of cells in administration group)/(number of cells in NIH3T3-IL17-IgG group - number of cells in NIH3T3 group) × 100. The results showed that the inhibition rates of the total number of infiltrating cells for each dose group (3, 10, and 30 mg/kg) of antibody hu31 were 50.0%, 56.7%, and 78.3%, respectively. The inhibition rate of the number of Gr1+ cells was calculated, and the results showed: the average Gr1+ cell inhibition rates for each dose group (3, 10, and 30 mg/kg) of antibody hu31 were 59.1%, 54.5% and 81.8%, respectively, and antibody hu31 could dose-dependently inhibit IL-17-induced total cell and inflammatory cell infiltration in mice.

## Claims

1. A pharmaceutical composition, **characterized in** comprising:
(1) a buffer; and
(2) an anti-IL-17A antibody or an antigen-binding fragment thereof; wherein the anti-IL-17A antibody or the antigen-binding fragment thereof comprises any one of the following (I) to (III):
(I) HCDR1, HCDR2 and HCDR3 with amino acid sequences as shown in SEQ ID NO: 1, SEQ ID NO: 2 and SEQ ID NO: 3, respectively, and LCDR1, LCDR2 and LCDR3 with amino acid sequences as shown in SEQ ID NO: 4, SEQ ID NO: 5 and SEQ ID NO: 6, respectively; or
(II) HCDR1, HCDR2 and HCDR3 with amino acid sequences as shown in SEQ ID NO: 7, SEQ ID NO: 8 and SEQ ID NO: 9, respectively, and LCDR1, LCDR2 and LCDR3 with amino acid sequences as shown in SEQ ID NO: 10, SEQ ID NO: 11 and SEQ ID NO: 12, respectively; or
(III) HCDR1, HCDR2 and HCDR3 with amino acid sequences as shown in SEQ ID NO: 13, SEQ ID NO: 14 and SEQ ID NO: 15, respectively, and LCDR1, LCDR2 and LCDR3 with amino acid sequences as shown in SEQ ID NO: 16, SEQ ID NO: 17 and SEQ ID NO: 18, respectively.

2. The pharmaceutical composition of claim 1, **characterized in that** the buffer is selected from an acetate buffer, a citrate buffer, a succinate buffer and a histidine buffer; preferably, the buffer is the histidine buffer; and preferably, the histidine buffer is a histidine-hydrochloride buffer.

3. The pharmaceutical composition of claim 1 or 2, **characterized in that** the buffer has a concentration of about 10-30 mM.

4. The pharmaceutical composition of any one of claims 1-3, **characterized in that** the buffer has a pH of about 5.0-6.5, preferably about 5.0-6.0, and more preferably about 5.5.

5. The pharmaceutical composition of any one of claims 1-4, **characterized in that** the pharmaceutical composition further comprises a stabilizer selected from one or more of arginine hydrochloride, proline, glycine, sodium chloride, mannitol, sorbitol, sucrose, maltose, xylitol and trehalose; preferably, the stabilizer is selected from arginine hydrochloride, a combination of arginine hydrochloride and mannitol, or a combination of arginine hydrochloride and sucrose.

6. The pharmaceutical composition of claim 5, **characterized in that** the stabilizer is sodium chloride at a concentration of about 30-200 mM; or the stabilizer is mannitol at a concentration of about 100-300 mM; or the stabilizer is sorbitol at a concentration of about 100-300 mM; or the stabilizer is sucrose at a concentration of about 100-300 mM; or the stabilizer is trehalose at a concentration of about 100-300 mM; or the stabilizer is arginine hydrochloride at a concentration of about 30-200 mM; or the stabilizer is proline at a concentration of about 100-300 mM; or the stabilizer is glycine at a concentration of about 100-300 mM; or the stabilizer is a combination of about 30-200 mM of sodium chloride and about 30-200 mM of mannitol; or the stabilizer is a combination of about 30-200 mM of sodium chloride and about 30-200 mM of sucrose; or the stabilizer is a combination of about 30-200 mM of arginine hydrochloride and about 30-200 mM of mannitol; or the stabilizer is a combination of about 30-200 mM of arginine hydrochloride and about 30-200 mM of sucrose.

7. The pharmaceutical composition of claim 6, **characterized in that** the stabilizer is arginine hydrochloride at a concentration of about 120 mM to about 170 mM; or a combination of about 60-120 mM of arginine hydrochloride and about 40-80 mM of sucrose; or a combination of about 60-120 mM of arginine hydrochloride and about 40-80 mM of mannitol.

8. The pharmaceutical composition of any one of claims 1-7, **characterized in that** the pharmaceutical composition further comprises a surfactant selected from polysorbate 80, polysorbate 20 or poloxamer 188.

9. The pharmaceutical composition of claim 8, **characterized in that** the surfactant has a concentration of about 0.01%-0.1%, preferably about 0.02%-0.08%, and more preferably about 0.02%-0.04%; preferably, the surfactant is about 0.01%-0.1%, preferably about 0.02%-0.08%, and more preferably about 0.02%-0.04% polysorbate 20.

10. The pharmaceutical composition of any one of claims 1-9, **characterized in that** the anti-IL-17A antibody comprises any one of the following (I) to (VI):
(I) a heavy chain variable region with an amino acid sequence as shown in SEQ ID NO: 19 and a light chain variable region with an amino acid sequence as shown in SEQ ID NO: 20; or
(II) a heavy chain variable region with an amino acid sequence as shown in SEQ ID NO: 21 and a light chain variable region with an amino acid sequence as shown in SEQ ID NO: 20; or
(III) a heavy chain variable region with an amino acid sequence as shown in SEQ ID NO: 21 and a light chain variable region with an amino acid sequence as shown in SEQ ID NO: 22; or
(IV) a heavy chain variable region with an amino acid sequence as shown in SEQ ID NO: 23 and a light chain variable region with an amino acid sequence as shown in SEQ ID NO: 24; or
(V) a heavy chain variable region with an amino acid sequence as shown in SEQ ID NO: 23 and a light chain variable region with an amino acid sequence as shown in SEQ ID NO: 25; or
(VI) a heavy chain variable region with an amino acid sequence as shown in SEQ ID NO: 26 and a light chain variable region with an amino acid sequence as shown in SEQ ID NO: 27.

11. The pharmaceutical composition of any one of claims 1-10, **characterized in that** the anti-IL-17A antibody comprises any one of the following (I) to (VI):
(I) a heavy chain amino acid sequence as shown in SEQ ID NO: 28 and a light chain amino acid sequence as shown in SEQ ID NO: 29; or
(II) a heavy chain amino acid sequence as shown in SEQ ID NO: 30 and a light chain amino acid sequence as shown in SEQ ID NO: 29; or
(III) a heavy chain amino acid sequence as shown in SEQ ID NO: 30 and a light chain amino acid sequence as shown in SEQ ID NO: 31; or
(IV) a heavy chain amino acid sequence as shown in SEQ ID NO: 32 and a light chain amino acid sequence as shown in SEQ ID NO: 33; or
(V) a heavy chain amino acid sequence as shown in SEQ ID NO: 32 and a light chain amino acid sequence as shown in SEQ ID NO: 34; or
(VI) a heavy chain amino acid sequence as shown in SEQ ID NO: 35 and a light chain amino acid sequence as shown in SEQ ID NO: 36.

12. The pharmaceutical composition of any one of claims 1-11, **characterized in that** the anti-IL-17A antibody or the antigen-binding fragment thereof has a concentration of about 20-200 mg/mL, preferably about 60-180 mg/mL, and more preferably about 80-150 mg/mL.

13. The pharmaceutical composition of any one of claims 1-12, **characterized in** comprising the components shown in any one of the following (1) - (12):
(1) (a) about 60 mg/mL-180 mg/mL of the anti-IL-17A antibody or the antigen-binding fragment thereof; (b) about 10-30 mM of the histidine buffer with a pH of about 5.0-6.0; (c) about 100-300 mM of sorbitol; (d) and about 0.01%-0.1% polysorbate 20 or polysorbate 80; or
(2) (a) about 60 mg/mL-180 mg/mL of the anti-IL-17A antibody or the antigen-binding fragment thereof; (b) about 10-30 mM of the histidine buffer with a pH of about 5.0-6.0; (c) about 30 mM to about 200 mM of arginine hydrochloride; (d) and about 0.01%-0.1% polysorbate 20 or polysorbate 80; or
(3) (a) about 60 mg/mL-180 mg/mL of the anti-IL-17A antibody or the antigen-binding fragment thereof; (b) about 10-30 mM of the histidine buffer with a pH of about 5.0-6.0; (c) a combination of about 30 mM to about 200 mM of sodium chloride and about 30-200 mM of mannitol; (d) and about 0.01%-0.1% polysorbate 20 or polysorbate 80; or
(4) (a) about 60 mg/mL-180 mg/mL of the anti-IL-17A antibody or the antigen-binding fragment thereof; (b) about 10-30 mM of the histidine buffer with a pH of about 5.0-6.0; (c) a combination of about 30 mM to about 200 mM of sodium chloride and about 30-200 mM of sucrose; (d) and about 0.01%-0.1% polysorbate 20 or polysorbate 80; or
(5) (a) about 60 mg/mL-180 mg/mL of the anti-IL-17A antibody or the antigen-binding fragment thereof; (b) about 10-30 mM of the histidine buffer with a pH of about 5.0-6.0; (c) a combination of about 30 mM to about 200 mM of arginine hydrochloride and about 30-200 mM of mannitol; (d) and about 0.01%-0.1% polysorbate 20 or polysorbate 80; or
(6) (a) about 60 mg/mL-180 mg/mL of the anti-IL-17A antibody or the antigen-binding fragment thereof; (b) about 10-30 mM of the histidine buffer with a pH of about 5.0-6.0; (c) a combination of about 30 mM to about 200 mM of arginine hydrochloride and about 30-200 mM of sucrose; (d) and about 0.01%-0.1% polysorbate 20 or polysorbate 80; or
(7) (a) about 80 mg/mL of the anti-IL-17A antibody or the antigen-binding fragment thereof; (b) about 20 mM of the histidine buffer with a pH of about 5.5; (c) a combination of about 50 mM of sodium chloride and about 140 mM of mannitol; (d) and about 0.02% polysorbate 20; or
(8) (a) about 150 mg/mL of the anti-IL-17A antibody or the antigen-binding fragment thereof; (b) about 30 mM of the histidine buffer with a pH of about 5.5; (c) about 135 mM of arginine hydrochloride; (d) and about 0.02% polysorbate 20; or
(9) (a) about 150 mg/mL of the anti-IL-17A antibody or the antigen-binding fragment thereof; (b) about 30 mM of the histidine buffer with a pH of about 5.5; (c) a combination of about 50 mM of sodium chloride and about 120 mM of sucrose; (d) and about 0.02% polysorbate 20;
(10) (a) about 150 mg/mL of the anti-IL-17A antibody or the antigen-binding fragment thereof; (b) about 30 mM of the histidine buffer with a pH of about 5.5; (c) a combination of about 90 mM of arginine hydrochloride and about 50 mM of mannitol; (d) and about 0.02% polysorbate 20;
(11) (a) about 150 mg/mL of the anti-IL-17A antibody or the antigen-binding fragment thereof; (b) about 30 mM of the histidine buffer with a pH of about 5.5; (c) a combination of about 90 mM of arginine hydrochloride and about 50 mM of sucrose; (d) and about 0.02% polysorbate 20;
(12) (a) about 150 mg/mL of the anti-IL-17A antibody or the antigen-binding fragment thereof; (b) about 15 mM of the histidine buffer with a pH of about 5.5; (c) a combination of about 90 mM of arginine hydrochloride and about 50 mM of sucrose; (d) and about 0.02% polysorbate 20.

14. The pharmaceutical composition of any one of claims 1-12, **characterized in** comprising the components shown in any one of the following (i) - (vi):
(i) (a) about 80 mg/mL to 150 mg/mL of the anti-IL-17A antibody or the antigen-binding fragment thereof; (b) about 10-30 mM of the histidine buffer with a pH of about 5.5-6.0; (c) about 200-280 mM of sorbitol; (d) and about 0.02%-0.04% polysorbate 20 or polysorbate 80; or
(ii) (a) about 80 mg/mL to 150 mg/mL of the anti-IL-17A antibody or the antigen-binding fragment thereof; (b) about 10-30 mM of the histidine buffer with a pH of about 5.5-6.0; (c) about 120 mM to about 170 mM of arginine hydrochloride; (d) and about 0.02%-0.04% polysorbate 20 or polysorbate 80; or
(iii) (a) about 80 mg/mL to 150 mg/mL of the anti-IL-17A antibody or the antigen-binding fragment thereof; (b) about 10-30 mM of the histidine buffer with a pH of about 5.5-6.0; (c) a combination of about 40 mM to about 100 mM of sodium chloride and about 40-150 mM of mannitol; (d) and about 0.02%-0.04% polysorbate 20 or polysorbate 80; or
(iv) (a) about 80 mg/mL to 150 mg/mL of the anti-IL-17A antibody or the antigen-binding fragment thereof; (b) about 10-30 mM of the histidine buffer with a pH of about 5.5-6.0; (c) a combination of about 40 mM to about 100 mM of sodium chloride and about 40-150 mM of sucrose; (d) and about 0.02%-0.04% polysorbate 20 or polysorbate 80; or
(v) (a) about 80 mg/mL to 150 mg/mL of the anti-IL-17A antibody or the antigen-binding fragment thereof; (b) about 10-30 mM of the histidine buffer with a pH of about 5.5-6.0; (c) a combination of about 60 mM to about 120 mM of arginine hydrochloride and about 40-80 mM of mannitol; (d) and about 0.02%-0.04% polysorbate 20 or polysorbate 80; or
(vi) (a) about 80 mg/mL to 150 mg/mL of the anti-IL-17A antibody or the antigen-binding fragment thereof; (b) about 10-30 mM of the histidine buffer with a pH of about 5.5-6.0; (c) a combination of about 60 mM to about 120 mM of arginine hydrochloride and about 40-80 mM of sucrose; (d) and about 0.02%-0.04% polysorbate 20 or polysorbate 80.

15. Use of the pharmaceutical composition of any one of claims 1-14 in the preparation of a medicament for the treatment or prevention of IL-17A-mediated diseases; preferably, the diseases include inflammatory diseases and autoimmune diseases, preferably selected from: arthritis, rheumatoid arthritis, ankylosing spondylitis, chronic obstructive pulmonary disease, systemic lupus erythematosus (SLE), lupus nephritis, asthma, multiple sclerosis, cystic fibrosis and psoriasis.
